(19) Europäisches Patentamt
European Patent Office
Office européen des brevets

(11) EP 1 854 348 A1

(12) EUROPEAN PATENT APPLICATION
published in accordance with Art. 158(3) EPC

(43) Date of publication:
**14.11.2007 Bulletin 2007/46**

(21) Application number: **06715166.2**

(22) Date of filing: **03.03.2006**

(51) Int Cl.:
***A01H 5/00*** *(2006.01)* ***C12N 15/09*** *(2006.01)*

(86) International application number:
**PCT/JP2006/304083**

(87) International publication number:
**WO 2006/117927 (09.11.2006 Gazette 2006/45)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **04.03.2005 JP 2005061344**

(71) Applicant: **SUNTORY LIMITED**
**Osaka-shi,**
**Osaka 530-8203 (JP)**

(72) Inventors:
- **ONO, Eiichiro**
  **Nagaokakyo-shi, Kyoto 617-0823 (JP)**
- **TANIGUCHI, Makoto**
  **Osaka, 596-0827 (JP)**
- **TARUI, Yutaka**
  **Osaka-shi, Osaka 546-0033 (JP)**
- **MIKI, Wataru**
  **Hyogo, 652-0803 (JP)**

(74) Representative: **Vossius & Partner**
**Siebertstrasse 4**
**81675 München (DE)**

# (54) TRANSFORMED PLANT CAPABLE OF PRODUCING POLYGLUTAMIC ACID

(57) A method for preparing a transgenic plant for producing polyglutamic acid and a transgenic method prepared by this method are provided. The method of the present invention comprises introducing a nucleic acid encoding the polyglutamic acid synthase A (pgsA), a nucleic acid encoding the polyglutamic acid synthase B (pgsB) and a nucleic acid encoding the polyglutamic acid synthase C (pgsC) into a plant.

EP 1 854 348 A1

## Description

TECHNICAL FIELD

This invention relates to a transgenic plant for producing polyglutamic acid and a method for preparing the same.

BACKGROUND ART

PGA (γ-polyglutamic acid), which is a viscous polymer produced by a bacterium belonging to the genus *Bacillus,* is a highly-polymerized substance having a single-stranded structure wherein D- and L-glutamic acids are bound via amide bond between amino group and carboxyl group at the γ-position.

Acid soil amounting to 40% on the earth at present is produced as follows. Namely, in an area at a relatively high temperature, long-lasting acidic rainfall induces eluviation of bases from the soil and thus the soil becomes acidic. In addition, the excessive use of chemical fertilizers and stripping of alkaline nutrients in crop harvesting are also causative to the acidification. In acidic soil, there arises the ionization of aluminum contained at a high level therein and the toxic aluminum thus formed inhibits the growth of plants.

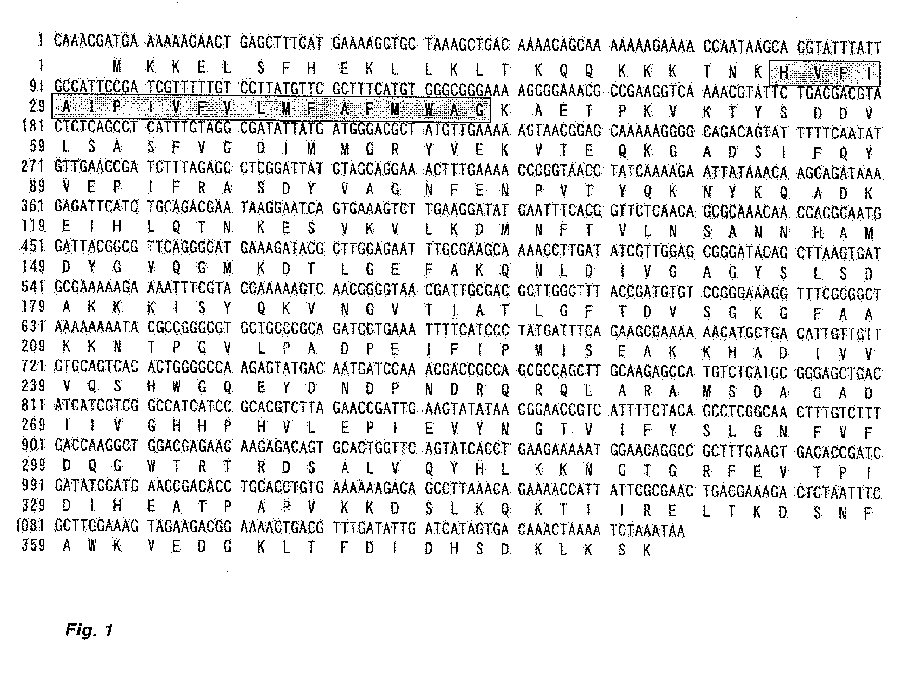

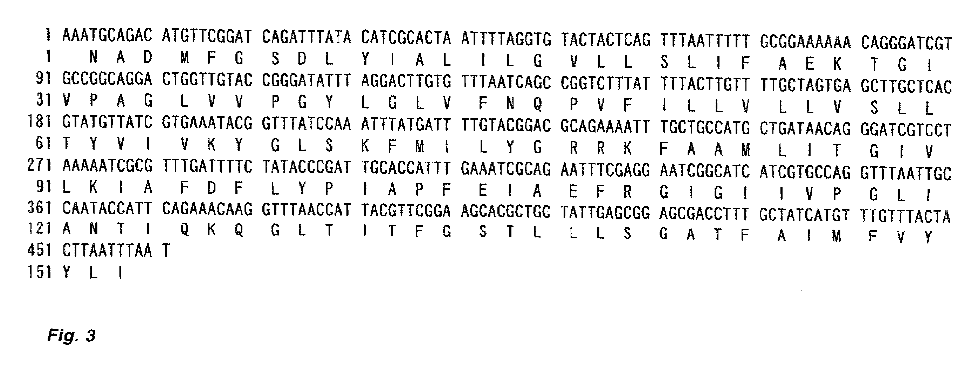

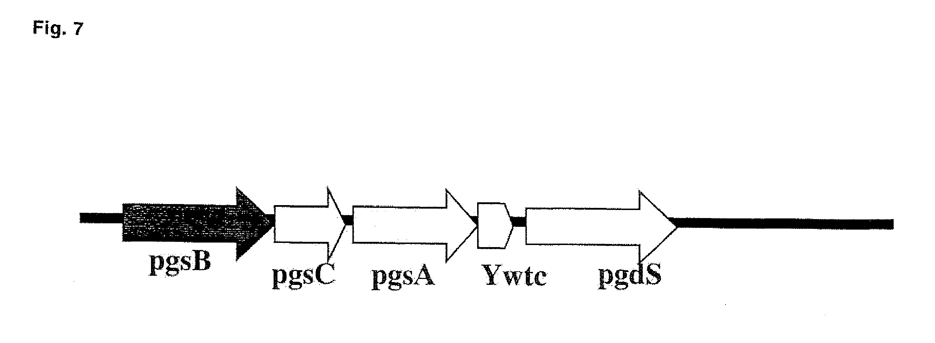

In *B. subtilis,* genes participating in the PGA synthesis are encoded by a single operon (Fig. 7). By using an *E. coli* transformation system, it has been clarified that PGA cannot be synthesized unless there are all of three genes pgsA, pgsB and pgsC (Fig. 1-3) required in PGA synthesis (Ashiuchi et al. *1999*). Based on the anticipated amino acid sequences, it is estimated that all of these three genes have transmembrane domains (Ashiuchi and Misono 2002). Moreover, it is proposed that pgsB has an ATP/GTP binding site motif characteristic to amido-ligase.

By analyzing isolated cell membrane fractions of *E. coli* transformants, it is proposed that gene products of pgsA, pgsB and pgsC are localized in the cell membrane of bacteria and have PGA synthesis activity (Ashiuchi et al. *2001*). To transfer a prokaryotic gene product into a eukaryote and allow it to sustain the normal function thereof, it is seemingly necessary that the gene product is integrated into the membrane structure. Although there have been known general methods for obtaining transgenic plants of eukaryotes, a specific gene can be rarely successfully expressed in a plant in an operable manner in practice. Thus, various creative efforts are needed therefor.

[Patent Publication No. 1]
JP-A-2003-420046
[Patent Publication No. 2]
JP-A-2003-432383
[Non-patent Publication No. 1]
Ashiuchi, M., Nawa, C., Kamei, T., Song, J.J., Hong, S.P., Sung, M.H., Soda, K. and Misono, H. (2001) Physiological and biochemical characteristics of poly-γ-glutamate synthetase complex of Bacillus subtilis, Eur. J. Biochem. 268: 5321-5328
[Non-patent Publication No. 2]
Horsch, R.B., Fry, J., Hoffmann, N., Neidermeryer, J., Rogers, S.G. and Fraley, R.T. (1988) Leaf disc transformation, Plant Mol. Biol. Mannual A5:1-9
[Non-patent Publication No. 3]
Johansen, L.K. and Carrington, J.C. (2001) Silencing on the spot, Induction and suppression of RNA silencing in the Agrobacterium-mediated transient expression system, Plant Physiol. 126:930-938
[Non-patent Publication No. 4]
Yamaguchi, F., Ogawa, Y., Kikuchi, M., Yuasa, K. and Motai, H. (1996) Detection of γ-polyglutamic acid (γ-PGA) by SDS-PAGE, Biosci. Biotech. Biochem. 60:255-258
[Non-patent Publication No. 5]
Yenofsky, R.L., Fine, M. and Pellow, J.W. (1990) A mutant neomycin phosphotransferase II gene reduces the resistance of transformants to antibiotic selection pressure, Proc. Natl. Acad. Sci. USA 87:3455-3439
[Non-patent Publication No. 6]
Ashiuchi et al. (1999) A poly-γ-glutamate synthetic system of Bacillus subtilis IFO 3336: gene cloning and biochemical analysis of poly-γ-glutamate produced by Escherichia coli clone cells; Bioche. Biophys. Res. Commun. 263, 387-393
[Non-patent Publication No. 7]
Ashiuchi and Misono (2002) Biochemistry and molecular genetics of poly-γ-glutamate synthesis, Appl. Microbiol. Biotechnol. 59, 9-14
[Non-patent Publication No. 8]
Shimonishi et al., Shin Seibutu Kagaku Jikken no Tebiki 3 (p.122-124, Kagaku Dojin, 1996)
[Non-patent Publication No. 9]
Hiei et al., Plant J., 6, p.271-282 (1994)
[Non-patent Publication No. 10]

Komari et al., Plant J., 10, p.165-174 (1996) [Non-patent Publication No. 11]
van Engelen et al., Transgenic Research 4, 288-290 (1995)
[Non-patent Publication No. 12]
Jefferson et al., (1987) GUS fusion:β-glucosidase as a sensitive and versatile gene fusion marker in higher plants, EMBO J. 6, 3901-3907
[Non-patent Publication No. 13]
Matsumoto and Machida (1990) Shokubutsu Keishitsu Tenkan-ho, Gendai Kagaku, 25-29
[Non-patent Publication No. 14]
Araki (2001) Genatsu Shinjun-ho ni yoru Keishitsutenkan, Saibo Kogaku (supplementary volume), Shokubutsu Saibo Kogaku Shirizu 15, 109-113
[Non-patent Publication No. 15]
Osumi (2001) Agurobakuteriumu Chokusetsu Chunyu-ho, Saibo Kogaku (supplementary volume), Shokubutsu Saibo Kogaku Shirizu 15, 105-108

DISCLOSURE OF THE INVENTION

PROBLEMS TO BE SOLVED BY THE INVENTION

An object of the present invention is to provide a method for preparing a transgenic plant for producing polyglutamic acid (PGA). The method of the invention comprises introducing a nucleic acid encoding the polyglutamic acid synthase A (pgsA), a nucleic acid encoding the polyglutamic acid synthase B (pgsB) and a nucleic acid encoding the polyglutamic acid synthase C (pgsC) into a plant. In practice, there has been obtained hitherto no plant in which pgsA, pgsB and pgsC genes are expressed in such a manner as exerting the PGA synthesis activity. Therefore, acquisition of a transgenic plant carrying PGA production genes introduced therein and thus having the function of synthesizing PGA contributes not only to the improvement of tolerance of the plant but also to the improvement of the environment by the plant per se. Furthermore, such a transgenic plant may have functions differing from the existing ones.
When PGA which is inherently secreted by a bacterium belonging to the genus *Bacillus* is secreted in the roots of a higher plant, it seems to bind to aluminum ion via ionic bond to form a water-soluble complex, thereby exerting an effect of preventing the absorption of aluminum ion from the roots. Since PGA would not inhibit the absorption of nutrients for plants such as nitrate nitrogen, phosphoric acid and potassium, it is expected that plant growth inhibition in acidic soil can be thus relieved. It is also known that PGA has a viscous nature and water holding ability. When PGA is synthesized in leaves, it is expected that the water holding ability of the plant is also enhanced, which may contribute to the development of a plant highly resistant to drought.
In one embodiment, the method according to the invention comprises infecting a plant with an Agrobacterium containing a vector inserted with one or more nucleic acids selected from a nucleic acid encoding the polyglutamic acid synthase A (pgsA), a nucleic acid encoding the polyglutamic acid synthase B (pgsB) and a nucleic acid encoding the polyglutamic acid synthase C (pgsC) to introduce the nucleic acids into the plant.
It is preferable that the introduction of the 3 nucleic acids, pgsA, pgsB and pgsC is performed by any one of the following steps a) - c):

a) infecting the plant with an Agrobacterium containing a single vector inserted with 3 nucleic acids, pgsA, pgsB and pgsC;
b) infecting the plant with three types of Agrobacterium each of them containing a distinct vector inserted with pgsA, pgsB or pgsC, respectively; or
c) infecting the plant with one or two types of Agrobacterium each of them containing a distinct vector inserted with pgsA, pgsB or pgsC, respectively to prepare transgenic plants having one or two nucleic acids selected from pgsA, pgsB and pgsC, and crossing the prepared transgenic plants.

The present invention further provides a transgenic plant for producing polyglutamic acid prepared by any of the methods as described above. The transgenic plant of the invention is preferably tobacco or *Arabidopsis thaliana.*

MEANS FOR SOLVING THE PROBLEMS

To solve the problems as discussed above, the present inventors conducted intensive studies and, as a result, successfully prepared a transgenic plant capable of producing polyglutamic acid (PGA) in vivo, thereby conceiving of the present invention.
First, three genes respectively encoding PGA synthases (pgsA, pgsB and pgsC) were taken out from a bacterium contained in *natto* (fermented soybeans) by PCR and electrophoresis, subjected to TA cloning and then integrated into a pGEM-T Easy vector. After sequencing, they were inserted into pGEM(T-HB)ΔSSS vectors at the EcoRI cut. Finally, they were inserted into a binary vector pBI121 at the BamHI/SacI cut. pBI121 was introduced into the Agrobacterium, which is a bacterium capable of integrating a T-DNA in plasmid into a plant infected therewith. By use of the Agrobacterium, the plant was then infected with the vectors by using the leaf disc method with the use of tobacco and the decompression-humectation method with the use of *Arabidopsis thaliana*, thereby preparing transgenic plants. Further, by using tobacco, the expression of these three genes was examined by the direct injection method, by which a transformation can be transiently expressed in somatic cells, and it was confirmed by the RT-PCR method that the introduced genes were expressed in the transgenic plants.

Furthermore, a construct in which these three genes pgsA, pgsB and pgsC were integrated into a single binary vector for plant transformation was prepared and introduced into a plant by the leaf disc method with the use of an Agrobacterium. As a result, a transgenic plant carrying all of these three genes introduced therein could be obtained.

Accordingly, the present invention aims at providing a method for preparing a transgenic plant for producing polyglutamic acid. The method according to the invention comprises introducing a nucleic acid encoding the polyglutamic acid synthase A (pgsA), a nucleic acid encoding the polyglutamic acid synthase B (pgsB) and a nucleic acid encoding the polyglutamic acid synthase C (pgsC) into a plant.

Nucleic acid encoding enzyme for synthesizing polyglutamic acid

The method according to the invention is characterized by comprising introducing 3 nucleic acids, i.e., a nucleic acid encoding the polyglutamic acid synthase A (pgsA), a nucleic acid encoding the polyglutamic acid synthase B (pgsB) and a nucleic acid encoding the polyglutamic acid synthase C (pgsC) into a plant. To produce polyglutamic acid in a plant body, it is necessary to introduce these three nucleic acids into a single plant body.

Nucleic acids usable in the invention involve genomic DNAs (including cDNAs corresponding thereto), chemically synthesized DNAs, DNAs having been amplified by PCR and combinations thereof.

The nucleic acid encoding pgsA preferably has the base sequence of SEQ ID NO:2. The nucleic acid encoding pgsB preferably has the base sequence of SEQ ID NO:4. The nucleic acid encoding pgsC preferably has the base sequence of SEQ ID NO:6. These base sequences of nucleic acids, which are obtained from *B. subtilis* as genes participating in PGA synthesis, are disclosed by, for example, Ashiuchi et al., 1999.

In some cases, more than one codons encode a single amino acid and this phenomenon is called degeneracy of the genetic code. Therefore, it is possible that a DNA sequence not completely identical with SEQ ID NO:2, 4 or 6 could encode a protein having an amino acid sequence that is completely identical with SEQ ID NO:1, 3 or 5. Such a mutant DNA sequence may be formed by a silent mutation (for example, one occurring in PCR amplification). Alternatively, it may be derived from a natural sequence by an intentional mutagenesis.

The pgsA gene preferably encodes the amino acid sequence of SEQ ID NO:1. The pgsB gene preferably encodes the amino acid sequence of SEQ ID NO:3. The pgsC gene preferably encodes the amino acid sequence of SEQ ID NO:5. However, the present invention is not restricted thereto. Namely, these genes may have amino acid sequences wherein one or more amino acid residues are deleted from, added to or substituted in these amino acid sequences. It is intended that any homologous proteins fall within the category, so long as the three types of the polyglutamic acid synthases A, B and C, together, have the function to produce polyglutamic acid. The characteristic of the present invention resides in introducing three nucleic acids into a plant for synthesizing polyglutamic acid. The base sequences of these three nucleic acids are not restricted to SEQ ID NOS:2, 4 and 6, so long as they encode amino acid sequences being equivalent in function to SEQ ID NOS:1, 3 and 5. The number of "amino acid mutations" preferably ranges from 1 to 20, more preferably from 1 to 10 and most desirably from 1 to 5.

The amino acid sequence encoded by the pgsA gene is at least about 70% identical, preferably about 80% or higher identical, more preferably 90% or higher identical, still more preferably 95% or higher identical and most desirably 98% or higher identical with the amino acid sequence represented by SEQ ID NO:1.

The amino acid sequence encoded by the pgsB gene is at least about 70% identical, preferably about 80% or higher identical, more preferably 90% or higher identical, still more preferably 95% or higher identical and most desirably 98% or higher identical with the amino acid sequence represented by SEQ ID NO:3.

The amino acid sequence encoded by the pgsC gene has at least about 70% identical, preferably about 80% or higher identical, more preferably 90% or higher identical, still more preferably 95% or higher identical and most desirably 98% or higher identical with the amino acid sequence represented by SEQ ID NO:5.

Amino acid percent identity may be determined by visual inspections and mathematical calculations. Alternatively, it is also possible to determine the percent identity between two protein sequences by comparing sequential data based on the algorithm according to Needleman, S.B. and Wunsch, C.D. (J. Mol. Biol., 48:443-453, 1970) with the use of GAP Computer Program available from UWGCG (University of Wisconsin Genetic Computer Group). Preferable default parameters in GAP Program include: (1) a scoring matrix blosum 62 reported by Henikoff, S. and Henikoff, J.G. (Porc. Natl. Acad. Sci. USA, 89:10915-10919, 1992); (2) gap weight: 12; (3) gap length weight: 4; and (4) no penalty for end gaps.

Moreover, use can be made of other programs for comparing sequential data commonly employed by a person skilled in the art. For example, percent identity can be determined by comparing with sequential data with the use of a BLAST program reported by Altschul et al., (Nucl. Acids. Res. 25., p.3389-3402, 1997). This program is available from the website of National Center for Biotechnology Information (NCBI) or DNA Data Bank of Japan (DDBJ) on the Net. Various conditions (parameters) for searching for homology by the BLAST program are mentioned in detail in the above-described site. Although the setting may be partly modified in some cases, the search is usually conducted with the use of the defaults.

In the method according to the present invention, it is preferred that the polyglutamic acid synthase A has the amino acid sequence of SEQ ID NO:1 or an amino acid sequence which is at least 70% identical with SEQ ID NO:1, the polyglutamic acid synthase B has the amino acid sequence of SEQ ID NO:3 or an amino acid sequence which is at least 70% identical with SEQ ID NO:3, and the polyglutamic acid synthase C has the amino acid sequence of SEQ ID NO:5 or an amino acid sequence which is at least 70% identical with SEQ ID NO:5., and that these three types of the polyglutamic acid synthases, A, B and C, together, have the function to produce polyglutamic acid.

It is well known by a person skilled in the art that even proteins having the same function would have different amino acid sequences depending on the original varieties thereof. Therefore, the pgsA, pgsB and pgsC genes respectively involve such homologs and mutants of the base sequences of SEQ ID NOS:2, 4 and 6, so long as they, together, have the function to produce polyglutamic acid.

The expression "to produce polyglutamic acid" as used herein means that in the case of introducing these three nucleic acids into a plant individual, this individual produces PGA in an amount with a statistically significant difference from a nontransgenic plant (non-transformed plant). Since PGA is not produced in plants inherently, the transgenic plant according to the invention is expected as exerting some advantageous effect compared with a non-transformed plant even in the case where it produces only a minor amount of PGA. When these three nucleic acids are introduced, a transformed plant prepared by the method according to the present invention produces PGA at a level 1.5 times, preferably 2 times, more preferably 3 times, still more preferably 5 times and most desirably 7 times, higher than in a nontransgenic plant (non-transformed plant).

In the present invention, preferable nucleic acids encoding the polyglutamic acid synthases A, B and C preferably include nucleic acids which are hybridizable with the base sequences of SEQ ID NOS:2, 4 or 6 under stringent conditions, i.e., under moderately or highly stringent conditions and the three polyglutamic acid synthases A, B and C, together, have the function to produce polyglutamic acid.

The expression "under stringent conditions" means being hybridizable under moderately or highly stringent conditions. More specifically speaking, the moderately stringent conditions can be easily determined by a person skilled in the art having common techniques based on, for example, DNA length. As mentioned in Sambrook et al., Molecular Cloning: A Laboratory Manual, 3rd Ed., Chap. 6-7, Cold Spring Harbor Laboratory Press (2001), fundamental conditions therefor include, concerning a nitrocellulose filter, using hybridization conditions for a washing solution for pre-hybridization (5xSSC, 0.5% SDS, 1.0 mM EDTA (pH8.0)) and about 50% formamide, 2xSSC-6xSSC at about 40 to 50°C (or another similar hybridization solution such as Stark's solution in about 50% formamide at about 42°C) and washing conditions at about 60°C with 0.5xSSC, 0.1% SDS. It is preferred that the moderately stringent conditions include hybridization conditions at about 50°C, 6xSSC. Similarly, the highly stringent conditions can be easily determined by a person skilled in the art based on, for example, DNA length. In general, these highly stringent conditions are defined as including hybridization and/or washing at a higher temperature and/or a lower salt concentration than in the moderately stringent conditions (for example, hybridization at about 65°C, 6xSSC to 0.2xSSC, preferably 6xSSC, more preferably 2xSSC, most desirably 0.2xSSC) and being accompanied by, for example, hybridization under such conditions as defined above and washing at about 68°C, 0.2xSSC, 0.1% SDS. In buffer solutions for hybridization and washing, SSC (1xSSC corresponding to 0.15M NaCl and 15 mM sodium citrate) may be substituted by SSPE (1xSSPE corresponding to 0.15M NaCl, 10mM $NaH_2PO_4$ and 1.25mM EDTA, pH 7.4). Washing is conducted for 15 minutes following the completion of the hybridization.

As being known by a person skilled in the art and will be described hereinbelow, moreover, it should be understood that the washing temperatures and the salt concentrations in washing solutions can be appropriately controlled, if necessary, so as to establish stringency at a desired level on the basis of the fundamental principle controlling the hybridization reaction and the stability of the double-strand (see, for example, Sambrook et al., (2001)). In the case of hybridizing a nucleic acid with a target nucleic acid having an unknown sequence, the length of the hybrid is assumed as being the length of the nucleic acid to be hybridized. In the case of hybridizing a nucleic acid with another nucleic acid having a known sequence, the length of the hybrid can be determined by locating the nucleic acid sequences in parallel and identifying one or more regions having the optimum sequential complementary. The hybridization temperature of a hybrid assumed as having less than 50 base pairs should be lower by 5 to 25°C than the melting temperature ($T_m$) of the hybrid. $T_m$ is determined by the following formulae.

Relating to a hybrid of less than 18 base pairs in length:

$$T_m\ (^{\circ}C)=2(\text{number of A+T bases})+4(\text{number of G+C bases})$$

Relating to a hybrid of more than 18 base pairs in length:

$$T_m\ (^{\circ}C)=8.15^{\circ}C+16.6(\log_{10}[Na^{+}])+41(\text{molar fraction}[G+C])-0.63(\%\ \text{formamide})-500/n$$

In the above formulae, N stands for the number of bases in the hybrid; and [$Na^+$] stands for the sodium ion concentration in the hybridization buffer (1xSSC: [$Na^+$]=0.165M). In a preferred case, the nucleic acids to be hybridized comprise each at least 8 nucleotides (more preferably at least 15 nucleotides, or still more preferably at least 20 nucleotides, at least 25 nucleotides, at least 30 nucleotides, at least 40 nucleotides and most desirably at least 50 nucleotides) or have a length amounting to at least 1% (more preferably at least 25%, or still preferably at least 50%, at least 70% and most desirably at least 80%) of the length of the nucleic acid with which it is to be hybridized and have sequential identity of at least 50% (more preferably at least 70%, or still preferably at least 75%, at least 80%, at least 85%, at least 90%, at least 95%, at least 97.5%, at least 99% and most desirably at least 99.5%) with the nucleic acid with which it is to be hybridized. As discussed above in greater detail, the sequential identity is determined by comparing the sequences of the nucleic acids to be hybridized which are located in parallel so as to maximize the overlaps and identity while minimize the gap between the sequences.

The nucleic acid percent identity may be determined by visual inspections and mathematical calculations. Although the percent identity between two nucleic acid sequences can be determined by visual inspections and mathematical calculations, it is preferred to determine the percent identity by comparing sequential data with the use of a computer program. As a typical and preferable example of such computer programs, Wisconsin Package Version 10.0 Program "GAP" available from UWGCG (University of Wisconsin Genetic Computer Group, Madison, Wisconsin) may be cited (Devereux et al., 1984, Nucl. Acids. Res. 12:387). By using this "GAP" Program, it is possible not only to compare two nucleic acid sequences or two amino acid sequences with each other but also to compare a nucleic acid sequence with an amino acid sequence. Preferable default parameters in the GAP Program include: (1) GCG implementation of a unary comparison matrix (containing a value of 1 for identities and 0 for non-identities) for nucleotides, and a weighted amino acid comparison matrix of Gribskov and Burgess, Nucl. Acids Res. 14:6745, 1986, as described by Schwartz and Dayhoff, eds., Atlas of Polypeptide Sequence and Structure, National Biomedical Research Foundation, pp. 353-358, 1979; or other comparable comparison matrices; (2) a penalty of 30 for each gap and an additional penalty of 1 for each symbol in each gap for amino acid sequences, or penalty of 50 for each gap and an additional penalty of three for each symbol in each gap for nucleotide sequences; (3) no penalty for end gaps; and (4) no maximum penalty for long gaps. An exemplary computer program for comparing sequences that is usable by a person skilled in the art is the BLASTN program available for use via the National Library of Medicine website http://www.ncbi.nlm.nih.gov/blast/b12seq/bls.html version2.2.7 or the UW-BLAST 2.0 algorithm. Standard default parameter settings for UW-BLAST 2.0 are described at the following Internet site: http://blast.wustl.edu. In addition, the BLAST algorithm uses the BLOSUM62 amino acid scoring matrix, and optional parameters that can be used are as follows: (A) inclusion of a filter to mask segments of the query sequence that have low compositional complexity (as determined by the SEG program of Wootton and Federhen (Computers and Chemistry, 1993); also see Wootton and Federhen, 1996, Analysis of compositionally biased regions in sequence databases, Methods Enzymol. 266: 554-71) or segments consisting of short-periodicity internal repeats (as determined by the XNU program of Clayerie and States (Computers and Chemistry, 1993)), and (B) a statistical significance threshold for reporting matches against database sequences, or E-score (the expected probability of matches being found merely by chance, according to the stochastic model of Karlin and Altschul (1990); if the statistical significance ascribed to a match is greater than this E-score threshold, the match will not be reported.); E-score threshold values are preferably, 0.5, or 0.25, 0.1, 0.05, 0.01, 0.001, 0.0001, le-5, le-10, le-15, le-20, le-25, le-30, le-40, le-50, le-75, or le-100 with an increase in preference.

Similarly, the nucleic acids according to the present invention involve nucleic acids having base sequences different from the base sequences of SEQ ID NOS:2, 4 and 6 due to the deletion, insertion or substitution of one or more bases but three types of the polyglutamic acid synthases, A, B and C, together, having the function to produce polyglutamic acid. Although the number of the bases to be deleted, inserted or substituted is not particularly restricted so long as the polyglutamic acid synthases have the function to produce polyglutamic acid, it preferably ranges from 1 to several thousands, more preferably from 1 to one thousand, still more preferably form 1 to 500, still more preferably from 1 to 200 and most desirably from 1 to 100.

A definite amino acid residue may be substituted by, for example, another residue having equivalent physicochemical characteristics. Examples of such a conservative substitution include the substitution of an aliphatic residue with another aliphatic one such as the substitution of Ile, Val, Leu or Ala by each other; the substitution of a polar residue by another polar one such as the substitution of Lys by Arg, Glu by Asp or Gln by Asn; or the substitution of an aromatic residue by another aromatic one such as the substitution of Phe, Trp or Tyr by each other. There have been known other conservative substitutions, for example, the substitution of a region having a hydrophobic nature as a whole by another region. Desired deletion, insertion or substitution can be made by a person skilled in the art by using well-known genetic engineering techniques via, for example, the site-specific mutagenesis method reported by Sambrook et al. (2001; see above) and so on.

In an embodiment of the method according to the present invention, therefore, the polyglutamic acid synthase A has the amino acid sequence of SEQ ID NO:1 or an amino acid sequence wherein one or more amino acid residues are deleted from, added to or substituted in SEQ ID NO:1, the polyglutamic acid synthase B has the amino acid sequence of SEQ ID NO:3 or an amino acid sequence wherein one or more amino acid residues are deleted from, added to or substituted in SEQ ID NO:3, and the polyglutamic acid synthase C has the amino acid sequence of SEQ ID NO:5 or an amino acid sequence wherein one or more amino acid residues are deleted from, added to or substituted in SEQ ID NO:5, and that these three types of the polyglutamic acid synthases, A, B and C, together, have the function to produce polyglutamic acid.

Introduction into plant

In the present invention, the method of introducing the nucleic acids A, B and C encoding polyglutamic acid synthases into a plant is not particularly restricted. Namely, use can be made of an appropriate publicly known method depending on the type of the plant. For the transduction by genetic engineering techniques, use may be made of any appropriate expression system. An expression vector contains the nucleic acids A, B and C encoding the polyglutamic acid synthases which are ligated in an operable matter to an appropriate transcription or translation regulatory sequence originating in, for example, a mammalian, microbial, viral or insect gene and can be introduced into a plant.

Examples of the regulatory sequence include a transcriptional promoter, operator or enhancer, a ribosome-binding site of an mRNA and sequences suitable for initiating and terminating transcription and translation. A nucleotide sequence is operably ligated in the case where the regulatory sequence functionally relates to a DNA sequence. In the case where a promoter nucleotide sequence regulates the transcription of a DNA sequence, therefore, the promoter sequence is operably ligated to the DNA sequence. In general, an expression vector carries a replication origin imparting a replication ability in a plant and a selection gene identifying a transformant having been incorporated therein. As a selection marker, use can be made of those commonly employed in the art by a conventional method. Examples thereof include antibiotic (for example, tetracycline, ampicillin, kanamycin or neomycin, hygromycin, spectinomycin and so on) tolerance genes.

If necessary, a sequence encoding an appropriate signal peptide (either natural or heterogeneous) may be incorporated into the expression vector. It is also possible that the DNA sequence of a signal peptide (a secretion leader) is fused in-frame to a nucleic acid sequence so as to first transcribe the DNA followed by the translation of mRNA into the fused protein containing the signal peptide.

As examples of the method for incorporating a DNA fragment of a gene into a vector such as a plasmid, methods reported by Sambrok, J., and Russell, D.W. (2001), Molecular Cloning: A Laboratory Manual, 3rd ed. (New York: Cold Spring Harbor Laboratory Press) may be cited. For convenience, use can be made of a commercially available ligation kit (for example, a product of Takara Shuzo Co., Ltd.). The recombinant vector (for example, a recombinant plasmid) thus obtained is introduced into a plant, i.e., the host cells.

The vector can be conveniently prepared by ligating a desired gene to a vector for recombination available in the art (for example, a plasmid DNA) by a conventional method. To transform a plant with the use of the nucleic acids according to the present invention, a vector for plant transformation is particularly useful. A vector for plants is not particularly restricted so long as being capable of expressing the gene in plant cells and thus producing the aimed protein. Examples thereof include pBI221 and pBI121 (both available from Clontech) and vectors obtained by modifying them. To transform a monocotyledon, in particular, use may be made of pIG121Hm, ptTOK233 (both reported by Hiei et al., Plant J., 6, 271-282(1994)), pSB424 (Komari et al., Plant J., 10, 165-174 (1996)) and so on.

A transgenic plant can be prepared by constructing a vector for transformation via the substitution by the nucleic acid fragment according to the present invention at the β-glucuronidase (GUS) gene site of the vector as described above and then introducing it into a plant. It is preferable that the vector for transformation contains at least a promoter, an initiation codon, a desired gene (the nucleic acid sequence of the polyglutamic acid synthase gene A, B or C or a part thereof), a termination codon and a terminator. Further, it may appropriately contain a DNA encoding a signal peptide, an enhancer sequence, the nontranslational regions in the 5' side and the 3' side of the desired gene, a selection marker region, etc. Although the promoter and the terminator are not particularly restricted so long as they can exert the functions in plant cells, examples of promoters showing constitutional expression include, in addition to the 35S promoter having been preliminarily incorporated in the above vector, promoters of actin and ubiquitin genes.

The pb112I vector employed in Example 2 herein carries cauliflower mosaic virus-origin 35S promoter capable of constitutionally expressing a gene introduced into plant cells. It was clarified by RT-PCR that the transferred pgsB had been transcribed into RNA. To screen the introduction of a vector into a plant, Kan-tolerance was imparted to pBI121 by a neomycin phosphotransferase II (NPTII) gene under the regulation by NOS promoter. Yenofsky et al. (1990) reported that the NPTII gene of pBI121 showed weak Kan-tolerance due to point mutation occurring therein. In the present invention, however, screening can be conducted with the use of 100 μg/ml of kanamycin and thus the pBI121 vector could be used without any problem.

Examples of a method of introducing a plasmid into host cells generally include the calcium phosphate method or the calcium chloride/rubidium chloride method, the electroporation method, the electroinjection method, a chemical treatment method with PEG or the like, the method of using a gene gun as reported by Sambrook, J. et al. (2001, see above).

Examples of a method of introducing a gene into a plant, in particular, include the methods of using Agrobacterium (Hiei et al., Plant J., 6, p.271-282 (1994); and Komari et al., Plant J., 10, p.165-174 (1996)), the electroporation method, the PEG method, the microinjection method, the microcollision method and so on. Any method may be used without restriction so long as a nucleic acid can be introduced into a desired plant thereby.

In the present invention, it is preferable to employ the Agrobacterium method. More specifically speaking, the plant is infected with an Agrobacterium containing a vector inserted with one or more nucleic acids selected from a nucleic acid encoding the polyglutamic acid synthase A (pgsA), a nucleic acid encoding the polyglutamic acid synthase B (pgsB) and a nucleic acid encoding the polyglutamic acid synthase C (pgsC) to introduce the nucleic acids into the plant.

In a more preferred embodiment, the three nucleic acids, pgsA, pgsB and pgsC are introduced by any one of the following steps a) - c):

a) infecting the plant with an Agrobacterium containing a single vector inserted with three nucleic acids, pgsA, pgsB and pgsC;

b) infecting the plant with three types of Agrobacterium each of them containing a distinct vector inserted with pgsA, pgsB or pgsC, respectively; or

c) infecting the plant with one or two types of Agrobacterium each of them containing a distinct vector inserted with pgsA, pgsB or pgsC, respectively to prepare transgenic plants having one or two nucleic acids selected from pgsA, pgsB and pgsC, and crossing the prepared transgenic plants.

The method according to the present invention involves a modification of the step b), e.g., infecting the plant with two types of Agrobacterium including one having a vector carrying two of pgsA, pgsB and pgsC (for example, pgsA+pgsB) inserted therein and the other one having a vector carrying the remainder (for example pgsC) inserted therein

The method according to the present invention involves a modification of the step c), e.g., infecting the plant with two types of Agrobacterium including one having a vector carrying two of pgsA, pgsB and pgsC (for example, pgsA+pgsB) inserted therein to prepare a transgenic plant having two of pgsA, pgsB and pgsC (for example, pgsA+pgsB), infecting a plant with an Agrobacterium having a vector carrying the remainder (for example pgsC) inserted therein to prepare another transgenic plant having the remainder (for example, pgsC), and then crossing the prepared transgenic plants. To prepare a transgenic plant having two types of nucleic acids, a vector containing the two nucleic acids may be used. Alternatively, a single plant may be infected with two Agrobacterium having different vectors from each other.

In the method according to the present invention, an embodiment with the use of the step a), i.e., using an Agrobacterium containing a single vector inserted with all of three nucleic acids, pgsA, pgsB and pgsC is preferred.

Common methods for inserting a gene into a plant with the use of an Agrobacterium are reported by Shimonishi et al., Shin Seibutsu Kagaku Jikken no Tebiki 3, (p.122-124, Kagaku Dojin (1996); Hiei et al., Plant J., 6, p.271-282 (1994); and Komari et al., Plant J., 10, p.165-174 (1996) and so on), though the invention is not restricted thereto.

First, a plasmid vector containing a nucleic acid to be inserted is constructed. As the plasmid vector, use can be made of, for example, SB11, pSB22, pBI121 (Jefferson et al., (1987) GUS fusion:β-glucosidase as a sensitive and versatile gene fusion marker in higher plants, EMBO J. 6, 3901-3907), pSPB176 (JP-A-2003-432383), pSPB541 (JP-A-2003-420046), pSFL203 (JP-A-2003-420046) and so on. Alternatively, a person skilled in the art can construct an appropriate vector on his/her own based on a plasmid vector such as one cited above. Next, *Escherichia coli* (for example, DH5α, JM109, MV1184, etc. each available form TAKARA) by using the recombinant vector carrying the nucleic acid insert.

By using the transgenic *Escherichia coli*, an Agrobacterium strain is subjected to triparential mating in accordance with, for example, the method of Ditta et al. (1980) preferably together with a helper *Escherichia coli* strain. As the Agrobacterium, it is possible to use, for example, *Agrobacterium tumefaciens* strains LBA4404/pSB1, LBA4404/pNB1, LBA4404/pSB3 and so on, though the invention is not restricted thereto. The plasmid maps of these strains are presented by Komari et al., Plant J., 10, p.165-174 (1996) as cited above and usable by a person skilled in the art by constructing a vector on his/her own. As the helper *Escherichia coli,* use can be made of, for example, DH1, pRK2013 (available from Clontech) and so on, though the invention is not restricted thereto. Furthermore, it is reported that *Escherichia coli* having pRK2073 is usable as the helper *Escherichia coli*, though it is less commonly employed.

In the present invention, a plant can be infected with the Agrobacterium by using a publicly known method. It is preferable to select an appropriate method in accordance with types of plants to be transformed from the group consisting of the leaf disc method Matsumoto & Machida (1990), Shokubutsu Keishitsu Tankan-ho, Gendai Kagaku, 25-29), the decompression-humectation method (Araki (2001), Genatsu Shinjun-ho ni yoru Keishitsu Tenkan, Saibo Kogaku (supplementary volume), Shokubutsu Saibo Kogaku Shirizu 15, 109-113) and the direct injection method (Osumi (2001) Agurobakuteriumu Chokusetsu Chunyu-ho, Saibo Kogaku (supplementary volume), Shokubutsu Saibo Kogaku Shirizu 15, 105-108). The leaf disk method is most desirable (Example 14).

In transformations with the use of tobacco conducted in Examples herein, the commonly known leaf disc method was employed. There has been reported that transgenic individuals of *Solanaceae*, *Brassicaceae, Fabaceae*, *Asteraceae*, *Malvaceae*, *Apiaceae*, *Salicaceae*, etc. can be relatively easily obtained by this method (Horsch et al., 1988). In Example herein, about 1.5 to 2 Kan-tolerant individuals were obtained per leaf section. Although abnormal cormus formation was observed in about 30% of regenerated individuals, these abnormal individuals showed lower growth speed than normal regenerated individuals and, therefore, could be eliminated at transplantation. Thus, it appears that the abnormal cormus formation would not affect the preparation of a transgenic plant. Such abnormal cormus formation was observed in the case of transforming by pBI121 having GUS gene as a control. This fact suggests that it is not a phenotype caused by a gene but morphological abnormality occurring at a certain rate in the regeneration from somatic cells. After transplanted into a fresh medium or a hormone-free medium, regenerated shoots showed almost the same growth morphology and speed as the control transformants. Namely, no change was observed depending on the introduction of the individual genes.

It has been reported that when an Agrobacterium was injected into open tobacco leaves, a gene was introduced and transiently expressed in mesophyllic cells (Johansen and Carrington, 2001). According to this report, it is suggested that in the case of injecting Agrobacterium having different genes, a single mesophyllic cell would be infected with multiple Agrobacterium, thereby enabling the achievement of the effects of multiple genes having been introduced therein.

In Examples herein, On the other hand, the decompression-humectation method was employed in introducing a gene into *Arabidapsis thaliana.* As a result, no morphological abnormality but some narrowing in leaf blade compared with the wild type was observed at the stage showing several rosette leaves.

<u>Confirmation of the expression of the genes encoding polyglutamic acid synthases A, Band C and/or the expression of polyglutamic acid</u>

In the transgenic plants prepared by the method according to the present invention, the expression of the genes encoding polyglutamic acid synthases A, B and C and/or polyglutamic acid can be confirmed by using a publicly known method.

The expression of the genes encoding polyglutamic acid synthases A, B and C can be confirmed by using a publicly known nucleic acid amplification method. Thus, the expression of preferably two or more types, more preferably all of the three types, of these genes can be confirmed.

Examples of the nucleic acid amplification reaction include reactions requiring temperature cycling such as the polymerase chain reaction (PCR) (Saiki R.K., et al., Science, 230, 1350-1354 (1985), the ligase chain reaction (LCR) (Wu D.Y., et al., Genomics, 4, 560-569 (1989)) and amplification based on transcription, and thermostatic reactions such as the strand displacement amplification (SDA (Walker G.T., et al., Proc. Natl. Acad. Sic. USA, 89, 392-396 (1992) ), the self-sustained sequence replication (3SR) (Guatelli J.C., Proc. Natl. Acad. Sci. USA, 87, 1874-1878 (1990)) and the Qβ replicase system (Lizardi et al., Bio Technology 6, p.1197-1202 (1988)) and so on. Moreover, it is also possible to employ, for example, the nucleic acid sequence based amplification (NASABA) reaction via the competitive amplification of a target nucleic acid and a mutant sequence described in European Patent No. 0525882. The PCR method may be cited as a preferable one.

The PCR method can be conducted by using primers having been prepared based on a sequence consisting of about 14 to about 30 bases selected from the code region or around it of the gene of the polyglutamic acid A, B or C and an RNA extracted from, for example, a leaf, a callus or the like of the transgenic plant as a template (RT-PCR). As an internal control, use can be made of the expression of a gene which is generally expressed over a wide scope in the natural type of the plant employed in the transformation (for example, tobacco ubiquitin gene, actin gene, etc.) as an indication of the expression. it is preferable that in the transgenic plant prepared according to the present invention, the gene of the polyglutamic acid synthase A, B or C is expressed at the RNA level of 1/10 or more, 1/5 or more, or 1/2 or more of the expression level of the internal control gene or almost comparable thereto, more preferably, almost comparable to the expression level of the internal control gene or twice or more or five times or more thereof.

The expression of polyglutamic acid in the transgenic plant prepared by the method according to the present invention can be confirmed by using a publicly known method. In the case where polyglutamic acid is secreted into a solution, it can be detected by the HPLC analysis of glutamic acid obtained by hydrolysis (Ashiuchi et al., 1999) or SDS-PAGE using methylene blue-staining (Yamaguchi et al., 1996).

In the present invention, it is expected that PGA is biosynthesized in tissues of the transgenic plant. To detect the biosynthesized PGA without resorting to the separation from cell wall components and proteins originating in the plants, it is therefore preferable to employ a detection method using an immunochemical technique. For example, Western blotting (dot blotting, in situ blotting, etc.) may be conducted with the use of a polyclonal antibody (antiserum) or a monoclonal antibody recognizing PGA, though the invention is not restricted thereto.

A polyclonal antibody (antiserum) recognizing PGA can be prepared by a conventional method with the use of PGA. For example, it can be obtained by immunizing an animal with an immunization antigen prepared by mixing PGA with RIBI adjuvant (MRL+TDM emulsion), Freund's complete adjuvant or Freund's incomplete adjuvant or an auxiliary agent such as alum. The animal to be immunized may be an arbitrary one selected from those commonly employed in the art, for example, mouse, rat, rabbit, goat, horse and so on. The immunization may be conducted either once or twice more at appropriate intervals (preferably from 1 to 5 weeks). The presence of the polyclonal antibody reacting with PGA can be examined by collecting the blood from the immunized animal and separating the serum from it.

In Example herein, RIBI adjuvant, which is less viscous and scarcely causes swelling, was mixed with PGA and subcutaneously injected into a mouse repeatedly at intervals of 2 weeks to thereby prepare a polyclonal antibody (Example 11).

Similarly, a monoclonal antibody recognizing PGA can be prepared by a publicly known method. To produce a monoclonal antibody, at least the following procedures are required: (a) formation of a hapten phthalate-polymer conjugate to be used as an antigen for the immunization; (b) immunization of an animal; (c) blood collection, assay and preparation of antibody-producing cells; (d) preparation of myeloma cells; (e) fusion of the antibody-producing cells with the myeloma cells and selective culture of hybridomas; (f) screening of the hybridomas producing the aimed antibody and cell cloning; (g) preparation of the monoclonal antibody by culturing the hybridomas or transplanting the hybridomas into an animal; and (h) evaluation of the reactivity of the thus prepared monoclonal antibody, etc.

Methods commonly employed for preparing monoclonal antibody-producing hybridomas are described in, for example, Hybridoma Techniques, Cold Spring Harbor Laboratory, 1980. The immunochemical detection of PGA with the use of an antibody can be carried out in accordance with a publicly known method.

In Example as will be described hereinafter, it was attempted to detect PGA by the dot blotting method. However, the antiserum thus obtained first showed only a low titer and thus PGA directly blotted on a nitrocellulose membrane could not be detected. To overcome this problem, we have developed a method wherein PGA is crosslinked with BSA by using a soluble carbodiimide EDC [1-ethyl-3-(3-dimethylaminopropyl)carbodiimide hydrochloride] and bound to a membrane via BSA.

The transgenic plant prepared according to the present invention produces and accumulates PGA in an amount with a statistically significant difference from a nontransgenic plant (non-transformed plant). Since PGA is not produced in plants inherently, the transgenic plant according to the invention is expected as exerting some advantageous effect compared with a non-transformed plant even in the of producing only a minor amount of PGA. For example, the production of PGA can be detected by using the immunochemical technique as described above.

The transgenic plant prepared according to the present invention produces PGA in an amount 1.5 times as much, preferably 2 times as much, more preferably 3 times as much, still more preferably 5 times as much and most desirably 7 times as much as the nontransgenic plant (non-transformed plant), though the invention is not restricted thereto.

<u>Transgenic plant</u>

Another object of the present invention is to provide a transgenic plant producing polyglutamic acid which is prepared by the method according to the invention.

The plant obtained by the method according to the present invention is not restricted in species. It is preferable to use a plant selected from the group consisting of tobacco, *Arabidopsis thaliana*, rice, soybean, bird's-foot trefoil, petunia, torenia and tomato. It is more preferable to select from the group consisting of tobacco, *Arabidopsis thaliana,* rice, soybean and bird's-foot trefoil. Tobacco or *Arabidopsis thaliana* is most desirable.

The transgenic plant in the present invention is not restricted in phase. It is preferably in the phase of adult, seed or callus and an adult is most desirable.

Preferably, the transgenic plant of the present invention produces a greening effect under the existence of aluminum ion, compared with a non-transformed plant, and/or produces the effect of relieving growth inhibition induced by aluminum ion (Example 15). While not wishing to be bound by theory, this may be because when PGA is secreted in the roots of the transgenic plant, it binds to aluminum ion via ionic bond to form a water-soluble complex, thereby preventing the absorption of aluminum ion from the roots.

BRIEF EXPLANATION OF THE DRAWINGS

[Fig. 1] Fig. 1 shows the base sequence of the pgsA gene and an anticipated amino acid sequence thereof.
[Fig. 2] Fig. 2 shows the base sequence of the pgsB gene and an anticipated amino acid sequence thereof.
[Fig. 3] Fig. 3 shows the base sequence of the pgsC gene and an anticipated amino acid sequence thereof.
[Fig. 4] Fig. 4 shows the results of Western blotting (supernatant) of tobacco leaf sections having the PGA synthases introduced therein by the direct injection method. The spots, beginning at the top, respectively show the results of the 1/100 sample, the 1/10 sample and the equivalent sample.
[Fig. 5] Fig. 5 shows the results of Western blotting (precipitate) of tobacco leaf sections having the PGA synthases introduced therein by the direct injection method. The spots, beginning at the top, respectively show the results of the 1/100 sample, the 1/10 sample and the equivalent sample.
[Fig. 6] Fig. 6 shows the results of Western blotting with the use of the crosslinked PGA-BSA.
[Fig. 7] Fig. 7 shows the structure of ORF on B. *subtilis* chromosome participating in the PGA metabolism. pgsA, pgsB and pgsC indicate regions encoding the PGA synthases A, B and C respectively. Ywtc means a gene having an unknown function. pgdS means PGA depolymerase.
[Fig. 8] Fig. 8 shows the expression of introduced genes in a transgenic tobacco plant having three types of pgs genes in such a manner allowing constitutional expression thereof. More specifically speaking, RT-PCR was conducted by using primers specific to individual genes to confirm the expression of these three types of pgs genes. As a result, it was confirmed that the three types of pgs genes were heterotopically expressed in transgenic tobacco (PGSox.) lines 10, 11 and 21. (NT: nontransgenic individual, NtUBQ: tobacco ubiquitin gene (internal standard gene)).
[Fig. 9] Fig. 9 shows the results of the confirmation of the PGS protein accumulation in crude leaf extracts of the transgenic tobacco lines 10, 11 and 21, in which the heterotopic expression of the three pgs genes had been confirmed in Fig. 8 by Western blotting.
[Fig. 10] Fig.10 shows the gemmation of the transgenic tobacco of the present invention gemmated under the existence of aluminum ion (0.04% $AlCl_3$).
[Fig. 11] Fig. 11 shows the chlorophyll content of the transgenic tobacco of the present invention gemmated under the existence of aluminum ion (0.04% $AlCl_3$).

EXAMPLES

Now, the present invention will be described in greater detail by reference to the following Examples. However, it is to be understood that the technical scope of the invention is not restricted to these Examples. Based on the statement in the present specification, changes and variations of the invention may be easily made by a person skilled in the art and all of these changes and variations fall within the technical scope of the present invention.

Example 1: Isolation of PGA synthase gene

In this Example, three types of genes (PGA synthase genes) pgsA, pgsB and pgsC for synthesizing PGA were isolated from *Bacillus natto*.
A commercially available natto bean was put into 1 ml of distilled water and vigorously stirred to give a suspension of *Bacillus natto*. To 1 μl of this suspension, 0.2 μl of cDNA polymerase mix, 1 μl of 10xpolymerase buffer, 1 μl of DNTPs (2 mM dATP, dCTP, dGTP and dTT each), and 0.5 μl of 50 μM forward and reverse primers respectively for pgsA, pgsB and pgsC (SEQ ID NOS:7 to 12) were added and the total volume was adjusted to 10 μl with distilled water. Next, PCR was performed for 2 minutes at 94°C once and for 33 cycles with each cycle consisting of 30 seconds at 94°C, 30 seconds at 59°C and 60 seconds at 72°C, followed by the incubation for 5 minutes at 72°C.
After adding 2 μl of 6xloading dye, the reaction mixture was electrophoresed on a gel plate (1XTAE 2%, Agarose S) and dipped in an ethidium bromide solution (0.2 μl/ml) for 30 minutes. Then the gel in a part detectable under UV was excised.
To the gel, 3 times as much an eluent solution (NaI: sodium iodide) was added and the mixture was allowed to stand at 50°C for 10 minutes. When the gel was completely dissolved, 5 μl of glass milk having been vortexed was added and the obtained mixture was allowed to stand for 30 minutes under slowly stirring. (In the case where the gel was in a small amount, the mixture may be shaken by hand for 5 minutes). After centrifuging at 15,000 rpm for 1 minute, the supernatant was discarded. Then, 180 to 200 μl of New Wash (a mixture of ethanol with water) was added and the mixture was pipetted. After centrifuging at 15,000 rpm for 1 minute, the supernatant was discarded and this procedure was repeated thrice. Next, the precipitate was dried at 50°C for 20 minutes. After adding 15 μl of distilled water, the mixture was centrifuged at 15,000 rpm for 1 minute and the supernatant was collected. This procedure was repeated to give 20 μl of a DNA solution.

Example 2: Integration of DNA fragment into sequencing vector

In this Example, an insert DNA (i.e., each of PgsA, PgsB and PgsC) purified from the agars gel were integrated into pGEM-T Easy Vector (manufactured by PROMEGA) for sequential analysis.

Each insert DNA had A overhung added thereto with the use of Taq polymerase. Thus, the vector should have a blunted end and a cohesive end (-T) attached thereto. Since pGEM-T Easy Vector available from PROMEGA had been thus treated, it was subjected to the following cloning as such.

To 0.5 μl of the vector DNA, 5 μl of 2xligation buffer, 3.5 μl of the insert DNA and 1 μl of T4 DNA ligase were added and the total volume was adjusted to 10 μl. After reacting by incubating at 4°C overnight, 250 μl of competent cells were added to the liquid reaction mixture thus ligated and lightly stirred. Then the mixture was allowed to stand on ice for 20 minutes. Next, a heat shock for 50 seconds at 42°C was loaded on it and then the mixture was immediately returned on ice and allowed to stand for 2 minutes. To the obtained sample, an SOC medium (2% Trypton, 5% yeast extract, 10 mM NaCl, 2.5 mM KCl, 20 mM $MgCl_2$, 20 mM glucose, pH 7.0) was added. The obtained mixture was stirred well and cultured at 37°C for 90 minutes while occasionally tumbling. An LB agar medium (diameter: 95 mm; 1.5% agar, 1% Bactotrypton, 0.5% yeast extract, 5 mM NaCl, pH 7.0, 0.200 μg/ml ampicillin, 0.4 mM IPTG, 50 μg/ml X-Gal) was inoculated with transformed *Escherichia coli* followed by incubation at 37°C overnight. The colonies thus grown on the plate was subjected to blue/white selection. To ensure the presence of the insert in white or pale blue colonies, PCR was conducted by using T7 and Sp6-2 (SEQ ID NOS:13 and 14) as primers.

Example 3: Analysis of the base sequence of insert DNA

In this Example, the base sequence of the PGA synthase gene integrated into the pGEM-T Easy Vector was determined. Detailed procedures therefor are as follows. After the completion of the PCR, colonies having electrophoresed and migrated as bands were suspended in 5 ml of the LB medium and cultured at 37°C overnight. Then the culture medium was centrifuged and the cells were harvested followed by the purification and sequencing of the DNA with the use of Mag Extractor-Plasmid-Kit.

First, 150 μl of the re-suspended liquor was added to *Escherichia coli* pellets and suspended well by pipetting. Next, 150 μl of a ligation buffer was added thereto and the mixture was stirred by tumbling the tube and then allowed to stand on ice for 5 minutes. After adding 120 μl of a neutralizing solution, the mixture was stirred by tumbling the tube and then allowed to stand on ice for 5 minutes. The mixture was centrifuged at 20,000xg for 5 minutes and the supernatant was transferred into another tube. 500 μl of an absorption solution and 30 μl of magnetic beads II were added to this tube and the mixture was stirred for 60 seconds. Subsequently, the tube was set on a magnetic stand and the magnetic beads were put toward the magnet. Then the tube was gently shaken together with the magnetic stand so as to shake down the solution sticking to the cap. Next, the supernatant was removed on the magnet stand in the same manner as described above. After repeating the procedure twice, 500 μl of 99% ethanol was added thereto. After stirring for 30 seconds, the mixture was centrifuged at 20,000xg for 1 minute. After removing the supernatant with the use of the magnet stand, the magnetic beads were dried at 37°C for 1 hour. Then, 50 μl of an eluent was added and the mixture was stirred for 60 seconds and centrifuged at 20,000xg for 5 minutes. Then 40 μl of the supernatant was collected by using the magnet stand and employed as a plasmid sample in the following step.

A sequence sample for sequencing was prepared by using Thermo Sequence Cycle Sequencing Kit (USB Corporation). To 5.5 μl of a plasmid sample, 1 μl of a reaction buffer, 0.6 μl of 10 mM T7-Cy5 primer (SEQ ID NO:13), 1 μl of 10 mM SP6-2Cy5.5 primer (SEQ ID NO:14) and 0.85 μl of Thermo Sequence DNA polymerase were added. After mixing well, the obtained mixture was pipetted in 2 μl portions into four 0.5 ml microtubes. Next, 2 μl portions of ddA Termination Mix, ddT Termination Mix, ddG Termination Mix and ddC Termination Mix were added respectively to the microtubes to give a total volume of 4 μl each. After mixing well and layering a mineral oil thereon, each mixture was preheated at 95°C for 2 minutes and then incubated for 60 cycles with each cycle consisting of 30 seconds at 95°C, 30 seconds at 60°C and 1 minute at 72°C. After the completion of the reaction, 3 μl of Loading Dye was added and the mixture was heated at 75°C for 2 minutes and thoroughly cooled on ice. The obtained matter was employed as a sample for sequencing and the sequence was read with the use of a sequencer (Long Read Tower Tm DNA Sequencer; Amersham).

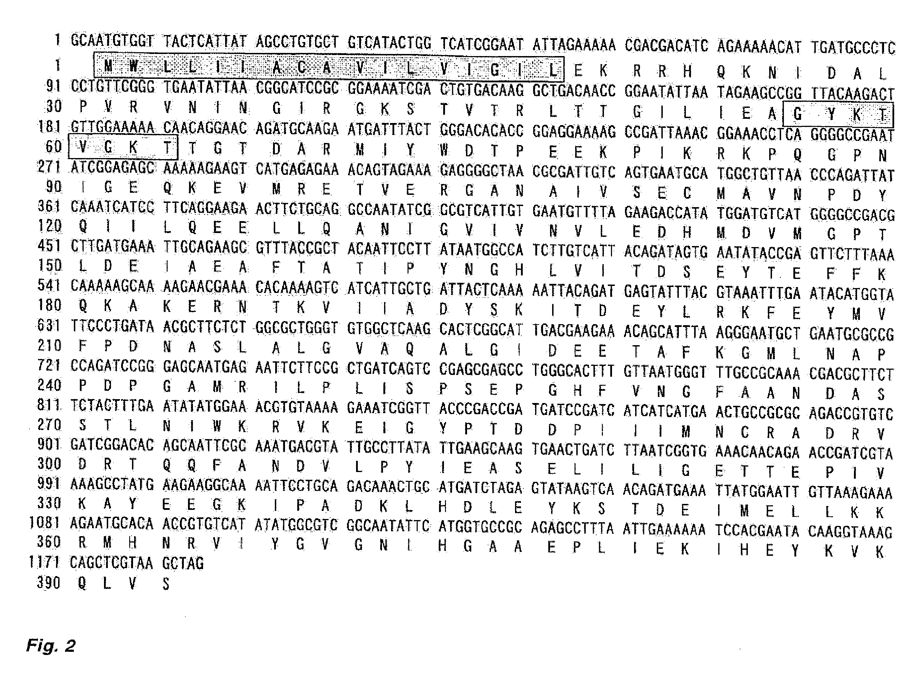

pgsA, pgsB and pgsC respectively had base sequences of 1149 bp, 1185 bp and 461 bp and encoded polypeptides having amino acid lengths of 380, 393 and 153 (Figs. 1 to 3). These data were almost completely identical with the sequences reported in *Bacillus subtilis* IFO3336 strain *(Bacillus natto)* (Ashiuchi et al., 1999). By using SOSUI algorithm (SOSUI:http//:sosui.proteome.bio.tuat.ac.jp), it was assumed that the polypeptides pgsA, pgsB and pgsC have 1, 1 and 5 transmembrane domains (the framed parts in Figs. 1 to 3). As the results motif searching (PORSITE PS00061. http://motif.genome.ad.jp), it was suggested that an ATP/GTP binding site motif exists in the 56- to 63-amino acids of pgsB (the underlined part in Fig. 2).

Example 4: Construction of vector for transformation (pBI121)

After the completion of the sequencing in Example 3, it was intended in this Example to construct vectors to be introduced into Agrobacterium to thereby transform a plant. Namely, the insert DNAs (PgsA, PgsB and PgsC) were individually transferred from the pGEM-T Easy Vector formed in Example 2 to pGEM(T-BH)ΔSSS vector and then to pBI121 vector.

First, 1 μl of a high buffer and 1 μl of a restriction enzyme EcoRI were added to 8 μl of the pGEM-T Easy Vector solution of Example 2 to give a total volume of 10 μl. Then the mixture was reacted at 37°C for 2 hours. For the ligation, the same insert DNAs as described above were extracted and purified. To 6 μl of an insert DNA having been cleaved with the restriction enzyme, 1 μl of 2xligation buffer, 1 μl of DNA ligase and 2 μl of pGEM(T-BH)ΔSSS, which had been similarly cleaved with EcoRI and dephosphorylated, were added to give a total volume of 10 μl. Then the mixture was incubated at 4°C overnight.

To 250 μl of competent cells, the liquid reaction mixture ligated above was added and thus transformation was conducted. After adding the SOC medium, the cells were inoculated into an LB agar medium and incubated at 37°C overnight. Colonies grown on the plate were subjected to the blue/white selection. For confirmation, PCR was conducted with the use of, as primers, T7 and a direction opposite to the site corresponding to any one of pgsA, pgsB and pgsC. Then, constructs having the 5' side of the sense sequence of the gene inserted into the T7 side were selected.

Subsequently, the corresponding colonies selected by the PCR and electrophoresis were cultured in the LB medium and plasmid DNA was extracted. To 40 μl of the DNA thus extracted, 5 μl of A low buffer and 5 μl of a restriction enzyme SacI were added to give a total volume of 50 μl. Next, the mixture was reacted at 37°C for 2 hours. To 50 μl of this sample, 10 μl of a high buffer and 5 μl of a restriction enzyme BamHI were further added and the total volume was adjusted to 100 μl. Then the mixture was reacted again at 37°C for 2 hours. Subsequently, the insert cleaved with SacI and BamHI was separated and DNA was extracted as described above. To 7 μl of the DNA cleaved with the restriction enzymes, 2 μl of 2xligation buffer, 1 μl of DNA ligase and 10 μl of pBI121 similarly cleaved with SacI and BamHI were added to give a total volume of 20 μl. Next, the mixture was incubated at 4°C overnight. After the completion of the reaction, 250 μl of competent cells were added to the liquid reaction mixture thus ligated followed by the addition of an SOC medium. The obtained mixture was inoculated into an LB agar medium containing an antibiotic kanamycin and incubated at 37°C overnight. For confirmation, colonies grown on the plate were subjected PCR with the use of, as primers, pBI-L and a direction opposite to the site corresponding to any one of pgsA, pgsB and pgsC. The PCR was conducted for 2 minutes at 94°C once, for 5 cycles with each cycle consisting of 30 seconds at 94°C, 30 seconds at 66°C and 60 seconds at 72°C, for 5 cycles with each cycle consisting of 30 seconds at 94°C, 30 seconds at 63°C and 60 seconds at 72°C, and for 28 cycles with each cycle consisting of 30 seconds at 94°C, 30 seconds at 59°C and 60 seconds at 72°C.

Example 5: Introduction into Agrobacterium

In the coexistence of a helper *Escherichia coli* for conjugative transfer, a vector in *Escherichia coli* was transferred into an Agrobacterium.

First, Escherichia coli colonies confirmed as having been transformed by the PCR and electrophoresis as described above were suspended in 3 ml of an LB liquid medium containing 100 μg/ml of kanamycin. After culturing overnight under shaking, a 200 μl portion was collected and centrifuged (9,000xg, 1 minute). After removing the supernatant, 200 μl of the liquid LB medium was added again and centrifugation was conducted. After repeating this procedure twice, the antibiotic was removed. To 20 μl of this sample, 20 μl of a helper Escherichia coli (DHI, pRK2013 strain) prepared in the same manner and 20 μl of an Agrobacterium suspension (LBA4404 strain) were added. The obtained mixture was stirred and then inoculated into an LB agar medium. After incubating at 28°C overnight, the colonies thus grown were suspended in 1 ml of the LB liquid medium. This sample was diluted 500-fold with the LB liquid medium and smeared on an LB agar medium containing 100 μg/ml of kanamycin and 100 μg/ml of streptomycin followed by incubation at 28°C over two night. Then the introduction of DNA was confirmed by PCR and electrophoresis.

EXAMPLE 6: Infection of plant by leaf disc method

By the leaf disc method using tobacco, a plant was infected with an Agrobacterium having a target DNA introduced therein. Different from prokaryotes, ORFs in eukaryotes are expressed as individual mRNAs and no protein can be synthesized in the form of operons. Thus, it was attempted to prepare a transgenic plant by introducing individual genes into different plants respectively and then crossing the obtained transgenic plants to give a plant having multiple genes.

First, a tobacco leaf section (about 1 cm x 1 cm) was floated on distilled water. With the back face of the leaf being upward, the section was placed on an MS agar medium (1xMS medium, 3% sucrose, 0.8% agar) containing 1 μg/ml of BA and 0.1 μg/ml of NAA. After sealing with a surgical tape, it was cultured at 25°C for 3 days. Then the section was put into a strainer and dipped in the Agrobacterium suspension of Example 5, which had been cultured under shaking at 28°C overnight in the LB liquid medium containing 100 μg/ml of kanamycin and 100 μg/ml of streptomycin, for 3 minutes. Next, the section was transferred to the MS agar medium containing 1 μg/ml of BA and 0.1 μg/ml of NAA. After sealing with a surgical tape, it was cultured in the dark for 3 days. Next, the section was transferred to the MS agar medium containing 1 μg/ml of BA, 0.1 μg/ml of NAA, 200 μg/ml of kanamycin and 300 μg/ml of carbenicillin. After sealing with a surgical tape, it was cultured for 2 to 3 weeks under illumination with 8 hours light/16 hours dark cycles.

After culturing the leaf section in the Kan-containing medium under the light/dark conditions for about 3 weeks, a large number of colonies were observed in the cut edge of the section and holes formed in the course of the operation. Since partly green colonies seemingly being Kan-tolerant appeared, the part was cut out and further cultured in a fresh Kan-containing medium.

Green individuals showing small shoot formation were selected and calluses and leaf sections around the shoot were cut. Next, they were transferred to the MS agar medium containing 1 μg/ml of BA, 0.1 μg/ml of NAA, 100 μg/ml of kanamycin and 300 μg/ml of carbenicillin again and sealed with a surgical tape. The individuals from which calluses had been cut were transplanted into tubes (diameter 39 mm) packed with the MS agar medium containing 100 μg/ml of kanamycin and 150 μg/ml of carbenicillin and cultured until rooting. Next, they were transplanted to soil and covered with a plastic sheet. After allowing to stand for several days, the plastic sheet was removed and the plants were grown thereafter, thereby harvesting seeds.

More specifically speaking, multiple green shoots were regenerated from calluses having pgsA and pgsB introduced therein. These shoots were transferred to a hormone-free medium to promote rooting. A shoot having pgsB introduced therein showed rooting. In the case of the pgsC introduction, the appearance of Kan-tolerant colonies and a structure seemingly being a tolerant shoot were confirmed. Although morphologically abnormal shoots were observed in the case of introducing pgsA and pgsB, such shoots were also observed in the control case of introducing pBI121. Thus, this phenomenon is considered as a transient abnormality which is generally observed in regenerating a plant from a callus. In fact, no morphologically abnormal organ was formed from shoots having been completely regenerated.

To examine pgsB gene expression in a tobacco callus considered as having the pgsB gene introduced therein by the leaf disc method due to the development of a Kan-tolerant callus, RT-OCR was conducted in accordance with the method of Example 10 as will be described hereinafter.

For reverse transcription, use was made of CDS-PRIM (SEQ ID NO:15) having oligo(T) complementary to poly (A) strand of mRNA. To confirm as not being PCR with the use of mixed DNAs as a template, reverse transcription was performed by using RNase-treated RNA as a negative control. The RNA concentrations used were 0.25 mg/ml, 0.025 mg/ml or 0.0025 mg/ml.

When amplified with the use of pgsB forward and reverse primers (SEQ ID NOS:9 and 10) as PCR primers, bands were observed in each case. This result was caused by the PCR amplification of DNAs contained in a relatively large amount. Therefore, PCR amplification was conducted by using, instead of pgsB forward and reverse primers (SEQ ID NOS:9 and 10), the pgsB forward primer and SMART-PR (SEQ ID NO:16) homologous with the 5' side of CDS-PRIM as primers. As a result, the reverse transcriptional product from the RNase-treated RNA was not amplified, while the reverse transcriptional product from the RNA without RNase treatment showed bands with dose-dependent signals. Based on these results, it was confirmed that mRNA of the pgsB gene was expressed in the tobacco leaf section and callus having the pgsB gene introduced therein.

Example 7: Infection of plant with decompression-humectation method

By the decompression-humectation method using *Arabidopsis thaliana,* a plant was infected with an Agrobacterium having a target DNA introduced therein. More specifically speaking, it was attempted to introduce a PGA synthase gene into *Arabidopsis thaliana* by humidifying inflorescence, infecting it with an Agrobacterium and directly introducing a gene into a cell in the early stage of the seed formation.

First, *Arabidapsis thaliana* seeds were sowed on soil covered with a mesh. When the plants grew to several centimeters in the scape height, they were made the same height by top pinching and small flower buds were cut off. Soil in pots was allowed to sufficiently absorb water and the plants were grown for about 1 week until stems began to extent again. Those having blossomed were cut off. Cells were collected from the Agrobacterium suspension of Example 5, which had been cultured in the LB liquid medium containing 100 μg/ml of kanamycin and 100 μg/ml of streptomycin at 28°C overnight under shaking, and suspended in a medium for suspension (1/2 MS salt, 1/2 Gamborg B5 vitamin, 5% sucrose, 0.5 g/l MES, BAP 10 ml/l. 0.02% Silwet L-77) to give $OD_{600}$ of 0.8. The suspension was pipetted into 400 ml beakers and the plants were dipped in the suspension by turning the pots upside down. Then the pots were put in a desiccator and allowed to stand under reduced pressure for 10 minutes. After removing the excessive suspension, the pots were placed on the side on a tray. Then a small amount of distilled water was dropped on the tray and the pots were covered and allowed to stand over day. After removing the cover, the pots were made upright and the plants were grown without irrigation for a week. Then, 100-fold diluted Hyponex was applied and the plants were grown for 2 to 3 weeks. Pods were collected and put into a bag containing silica gel. Then seeds in the pods were harvested. 80 to 100 μl of dry seeds were put in 70% ethanol for 2 minutes and then in 5% Antiformin-0.1% Tween 20 for 15 minutes. After washing with sterilized water 3 to 5 times, the seeds were suspended in a 9 ml of 0.1% aqueous solution of agar (sterilized). Next, the suspension was smeared on a selection medium (MS salt, Gamborg B5 vitamin, 1% sucrose, 0.5 g/l MES, 0.8% agar) containing 100 μg/ml of kanamycin and 100 μg/l of carbenicillin. After sealing with a surgical tape, it was allowed to stand at 4°C for 1 week. Then, it was cultured at 24°C for about 1 week and tolerant plants were transplanted into the same selection medium as described above. When 5 or 6 true leaves had opened, the plants were transplanted into soil and seeds were harvested.

From seeds obtained from the plant into which the introduction of pgsC had been attempted, young seedlings having green cotyledon/true leaves was obtained on the Kan-containing medium. These individuals are now growing in soil containing vermiculite. Concerning pgsA and pgsB, on the other hand, it is now attempted to select tolerant plants.

Example 8: Infection of plant by direct injection method

By the direct injection method using tobacco, a plant was infected with an Agrobacterium having a target DNA introduced therein in this Example. It is known that an when Agrobacterium having been cultured in the presence of syringone is injected into a tobacco leaf, mesophyllic cells are infected with it and shows the transient expression of a transferred gene (Johansen and Carrington 2001). Since it is suggested that genes of different types can be simultaneously introduced and expressed by infecting plant cells with multiple Agrobacterium by this method, attempts were made in the present study to examine whether or not pgsA, pgsB and pgsC could be transiently and simultaneously expressed via the simultaneous infection with Agrobacterium respectively carrying these genes.

First, cells of the Agrobacterium suspension of Example 5, which had been cultured under shaking in the LB liquid medium containing 100 μg/ml of kanamycin, 100 μg/ml of streptomycin and 20 μM of acetosyringone at 28°C overnight, were collected and re-suspended in 10 mM $MgCl_2$, 10 mM MES (pH5.7) and 150 μM acetosyringone to give $OD_{600}$ of 0.5. After incubating at ordinary temperature for 3 hours, tobacco leaves were perforated with an injection needle the suspension was injected into tobacco mesophyll with a 1 ml tuberculin syringe. After 3 days, the leaves were cut off, collected, freeze-dried at -80°C and then employed in RNA extraction as will described hereinafter. Moreover, these leaves were carefully ground in a mortar cooled with liquid nitrogen and put into a 15 ml tube. Next, 3 ml of 100 mM MES (pH4.5) was added thereto. The obtained mixture was transferred into an Eppen tube and centrifuged at 2.000xg for 10 minutes at 4°C to thereby separate into a supernatant and a precipitate. The supernatant was preserved at -20°C. To the precipitate, 150 μl of 100 mM MES (pH4.5) was added. The obtained mixture was stored at -20°C and employed in the following PGA detection.

Tobacco leaves were perforated with an injection needle and combinations of Agrobacterium (pgsA+pgsB, pgsB+pgsC, pgsC+pgsA and pgsA+pgsB+pgsC) were injected therein with the use of a syringe. After 3 days, RNAs were extracted from the leaves and the mRNA transcriptions from the introduced genes were confirmed by TR-PCR. As PCR primers, use was made of forward primers of pgsA, pgsB and pgsC (SEQ ID NOS:7, 9 and 11 respectively) and SMART-PR (SEQ ID NO:16) having homology with a part of the primer employed in the reverse transcription. Smears were merely observed in electrophoresis and no band assignable to an assumed molecular weight could be obtained.

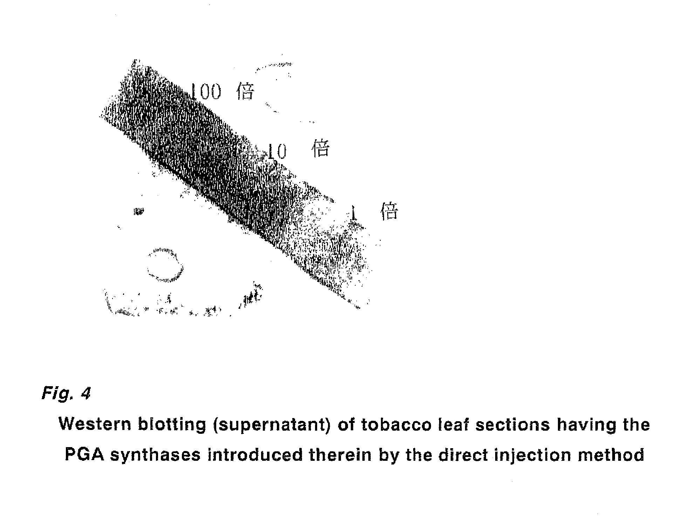

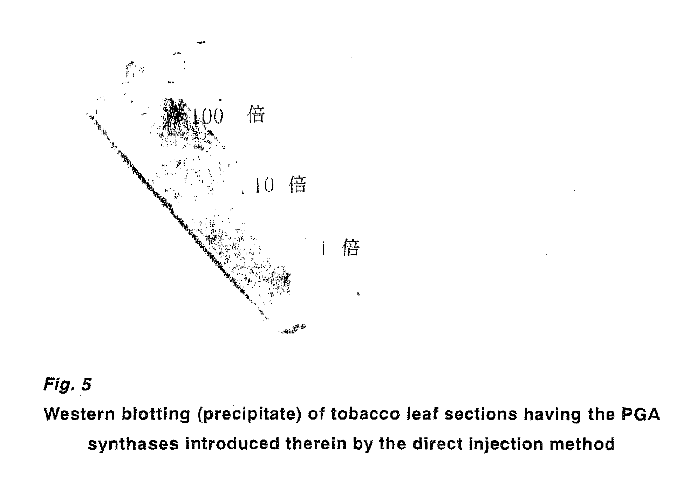

By using anti-PGA polyclonal antiserum prepared in Example 11 as will be described hereinafter, the presence or absence of PGA production was examined (Figs. 4 and 5). The antiserum first prepared had only a low titer and thus PGA directly blotted on a membrane could not be detected by it. Thus, PGA in tobacco leaves was covalently bound to BSA with the use of EDC by taking advantage of the property that BSA would aggregate together with various proteins (including PGA to be detected) due to crosslinkage by EDC. Subsequently, the mixture was divided into a supernatant and a precipitate by centrifugation.

Each of the crosslinked samples was spotted on a membrane in a dilution series of 1-fold, 10-fold and 100-fold. In the immunochemical detection by using the ABC method, slight color development was observed in the 1-fold and 10-fold samples of the precipitate.

Example 9: RNA extraction from transgenic plant

To examine the RNA expression of the introduced genes, RNAs were extracted from the transgenic individuals of Examples 6 to 8

A callus or a leaf of a transgenic individual was carefully ground in a mortar cooled with liquid nitrogen. Next, 3 ml of an RNA extraction buffer (100 mM NaCl., 50 mM Tris-HCl (pH9.0), 10 mM EDTA, 3% SDS) and 1 ml of phenol (0.5 M Tris-HCl, saturated at pH8.0) were added and the mixture was shaken at room temperature for 20 minutes. Next, 1 ml of a mixture of chloroform:isoamyl alcohol (v/v, 24:1) was added thereto. After vigorously suspending, the suspension was centrifuged at 2,000xg for 20 minutes at room temperature. The aqueous phase (the upper phase) was transferred into a sterilized capped centrifugation tube while paying much attention not to suck the protein precipitate in the intermediate layer. Then 1/3 times as much 10 M LiCl and 200 μl of 50 mM EDTA (pH8.0) were added and the mixture was allowed to stand overnight at 20°C. After centrifuging again at 2,000xg for 60 minutes at 4°C, the supernatant was completely removed. To the precipitate, 2 M LiCl and 1 ml of 50 mM EDTA (pH8.0) were added and the obtained mixture was allowed to stand for 1 hour at -20°C. After centrifuging again at 2,000xg for 60 minutes at 4°C, the supernatant was completely removed. Next, 400 μl of sterilized water was added and the precipitate was dissolved therein. The solution thus prepared was transferred into a 1.5 ml microtube. In accordance with the phenol-chloroform extraction method, nucleic acids were purified. After adding 20 μl of 3 M sodium acetate (pH5.2) and 1 ml of 99% ethanol and mixing well, the mixture was allowed to stand overnight at -20°C. Then it was centrifuged at 15,000 rpm for 30 minutes at 4°C. The precipitate thus obtained was washed with 70% ethanol. After completely removing the ethanol, the precipitate was air-dried and 50 μl of sterilized water was added so as to dissolve the precipitate therein.

Example 10: RT-PCR method for examining RNA expression

To examine the expression of the introduced genes, RT-PCR was conducted with the use of the RNAs extracted above as a template.

To quantify RNAs in the solution, 1 μl of the solution was dissolved in 1 ml of sterilized water and the absorbance was measured with a spectrophotometer. By referring the RNA content at $A_{260}$=20 as 1 mg/ml, the concentration was calculated.

For the reverse transcription of the RNA, 1 μl of CDS prim (SEQ ID NO:15) was added to 2 μl (5 mg/ml) of the RNA. After adjusting the total volume to 5 μl, the mixture was lightly centrifuged and incubated at 95°C for 5 minutes. After cooling to 50°C, 10 μl of dNTPs (dATP, dCTP, dGTP and dTTP, each 2 mM), 4 μl of 5xRT buffer, 0.5 μl of an RNA inhibitor and 1 μl of a transcriptase ReverTraAce (TOYOBO) were added and the obtained mixture was incubated at 50°C for 60 minutes.

To a 1 μl portion of the sample thus obtained, 0.05 μl of rTaq polymerase, 1 μl of 10xpolymerase buffer, 1 μl of dNTPs (dATP, dCTP, dGTP and dTTP, each 2 mM), 1 μl of 25 mM $MgCl_2$, 0.5 μl of 10 μM forward primers of pgsA, pgsB and pgsC, and 0.5 μl of 10 μM SMART-PR 2M (SEQ ID NO:16) were added. After adjusting the total volume to 10 μl with sterilized water, PCR was performed for 2 minutes at 94°C once and for 33 cycles with each cycle consisting of 30 seconds at 94°C, 30 seconds at 59°C and 90 seconds at 72°C. Then it was electrophoresed in the same manner as described above and the appearance of bands was confirmed by UV.

Example 11: Construction of polyclonal antibody using mouse

As an antibody against PGA, a polyclonal antibody was constructed by using a mouse.

2 ml of PBS (100 mM sodium phosphate, 150 μM NaCl, pH7.2) was added to RIBI adjuvant (MRL+TDM emulsion) having been solidified to dryness. The mixture was rotated on a rotary disc for several minutes for sufficiently suspending. To 200 μl of the adjuvant, PGA (10 mg/ml in PBS) was added at a ratio of 1:1 (v/v). At first, 100 μl portions of the mixture were subcutaneously injected into 2 sites in the abdomen of the mouse disinfected with 70% ethanol. Next, 150 μl/site of the mixture was subcutaneously injected at intervals of 2 weeks. One week after the third administration, the tail of the mouse was cut off and the blood was collected. Then the blood was allowed to stand for 30 minutes for coagulation and centrifuged at 10,000 rpm for 10 minutes. The supernatant was taken and mixed with the equivalent amount of PBS. The obtained mixture was employed as antiserum in the following experiments.

Example 12: Preparation of PGA-BSA bound protein

To bind PGA to a nitrocellulose membrane in Western blotting, a PGA-BSA bound protein was prepared by using EDC.

2 mg of BSA dissolved in 200 μl of a binding buffer (0.1 M MES, pH4.5) was mixed with 2 mg of PGA dissolved in 500 μl of a binding buffer.

After adding 100 μl of 10 mg/ml EDC [1-ethyl-3-(3-dimethylaminopropyl)carbodiimide hydrochloride], the mixture was reacted at ordinary temperature for 2 hours. Then, it was centrifuged at 15,000 rpm for 1 minute and the supernatant was discarded. After mixing the binding buffer by pipetting, centrifugation was conducted again and the supernatant was discarded. This procedure was repeated thrice. Then, 1 ml of PBS was added and the mixture was suspended well. The obtained product was employed as the PGA-BSA bound protein in the experiment of Example 13.

Example 13: Immunological detection of PGA using mouse serum

Dot blotting was conducted by the ABC method with the use of the serum of Example 11 and the bound protein of Example 12. Namely, natural PGA employed as an antigen was subcutaneously injected together with an adjuvant into a mouse repeatedly and it was attempted to detect the antigen-antibody reaction of the anti-PGA serum by dot blotting.

First, a nitrocellulose membrane was cut into a piece of an appropriate size. The PGA-BSA bound protein diluted 1-fold, 10-fold and 100-fold with PBS was spotted on the membrane and allowed to stand until dried. After UV-irradiating for 30 seconds, the membrane was put into a membrane bag. After adding 500 μl of saturated casein having been lightly centrifuged, the bag was sealed and shaken at room temperature for 30 minutes. Then casein was removed from the bag and the serum diluted 200-fold with casein was added. After sealing, the bag was shaken at room temperature for 30 minutes. Next, the membrane was taken out from the bag and washed with Tapper containing 3 ml of PBST (0.1% Tween in PBS) for 5 minutes thrice. Next, the membrane was put into a fresh bag and 500 μl of a 0.6% secondary antibody (a biotinylated universal antibody constructed in a horse) diluted with a casein solution was added. After sealing, the bag was shaken at room temperature for 30 minutes. Next, the membrane was taken out from the bag and washed with Tapper containing 3 ml of PBST for 5 minutes thrice. Next, the membrane was put into a fresh bag and a mixture, which had been preliminarily prepared by adding 3 μl of avidin (ABC-AP REAGENT A) and 3 μl of biotinylated alkali phosphatase (ABC-AP REAGENT B) to 500 μl of casein and allowing to stand for 30 minutes, was added thereto. After sealing, the bag was shaken at room temperature for 30 minutes. Next, the membrane was taken out from the bag and washed with Tapper containing 3 ml of PBST for 5 minutes thrice. Next, the membrane was washed with TAPPER impregnated with 100 mM Tris-HCl for 5 minutes. Then the membrane was put into a fresh bag and 500 μl of a 1.2% (v/v) substrate (BCIP, NBT, $MGCl_2$) diluted with 100 mM Tris-HCl was added thereto. After sealing, the bag was allowed to stand in dark overnight for color-development.

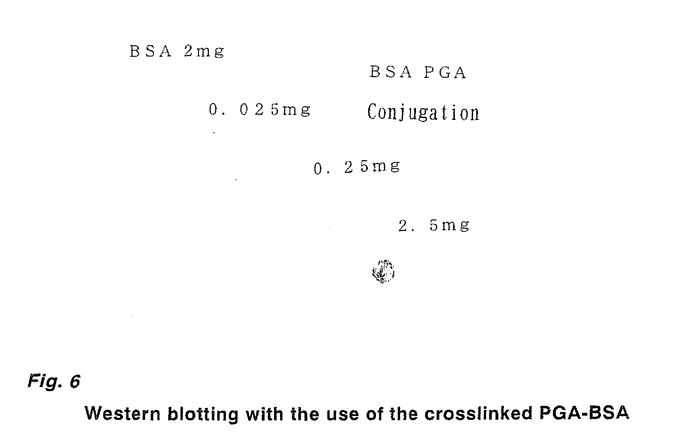

Although an experiment was repeated several times for detecting by spotting PGA directly on the membrane, so signal was detected. As the results of an examination by the amide black-staining, it was considered that the above phenomenon arose since PGA was separated from the membrane due to the low titer of the antibody at first and the weak binding of PGA to the membrane. Thus, PGA was covalently bound to BSA and bound to the membrane via BSA. Thus, a strong signal was observed in the 2 μl spot of the BSA complex containing 2.5 mg/ml of PGA (Fig. 6). An extremely weak signal was observed in 0.25 mg/ml PGA, whereas no signal was observed in 0.025 mg/ml PGA or 2 mg/ml BSA employed as a negative control.

The primary antiserum employed first was obtained in the ninth week after the antigen administration. No signal was obtained from the serum before this point. Since PGA is a soluble and relatively simple polypeptide, it was considered that the titer thereof was elevated only slowly. By continuously administering the antigen (PFA) to obtain an antiserum having a higher reactivity, an antiserum having a high titer was obtained. Thus, a signal could be detected on the membrane even in the case of not crosslinking PGA with BSA.

Example 14: Analysis of functions of pgs genes in plant cells

To clarify the functions of the three types of polyglutamic acid synthase genes (pgsA, pgsB and pgsC) as described above in plant cells, a transgenic tobacco plant (N. tabaccum) constitutionally expressing these three pgs genes was prepared.

Using 0.1 ng portions of the plasmid vectors (pGEM-T EASY, Promega) containing respective pgs genes that were prepared in Example 2, amplification was conducted by PCR under the following conditions. A PCR reaction mixture (50 μl) comprised 0.1 ng of the plasmid containing each of the pgs genes, 1xKOD plus buffer (TOYOBO), 0.2 mM dNTPs, 0.4 pmol/μl portions of primers having restriction enzyme site (SEQ ID NOS:17 to 22), 1 mM $MgSO_4$ and 1 U of KOD plus DNA polymerase. After reacting at 94°C for 5 minutes, the reaction was conducted for 30 cycles with each cycle consisting of 1 minute at 94°C, 1 minute at 55°C and 2 minutes at 72°C. The amplified fragments containing the full length of respective pgs genes were inserted into the multicloning site of pCR-blunt II TOPO vector (INVITROGEN) to give TOPO-pgsA (pSPB2667), TOPO-pgsB (pSPB2668) and TOPO-pgsC (pSPB2669). The base sequences of these subcloned pgs genes were determined by the primer walking method and it was confirmed that PCR had caused no mutation.

SEQ ID NO:17: (BamHI-pgsA-FW)
5'-agg gga tcc acg atg aaa aaa gaa ctg agc ttt cat gaa-3'
SEQ ID NO:18: (SacI-pgsA-FW)

5'-agg gag ctc tta ttt aga ttt tag ttt gtc act atg atc a-3' SEQ ID NO:19: (BamHI-pgsB-FW)
5'-agg gga tcc gca atg tgg tta ctc att ata gcc tgt gct-3'
SEQ ID NO:20; (XhoI-pgsB-RV)
5'-agg ctc gag cta gct tac gag ctg ctt tac ctt gta tt-3'
SEQ ID NO:21:(KpnI-pgsC-FW)
5'-agg ggt acc gac atg ttc gga tca gat tta tac atc gca-3'
SEQ ID NO:22:(BamHI-pgsC-RV)
5'-agg gga tcc tta aat taa gta gta aac aaa cat gat-3'

A binary vector pSPB2672 was obtained by attaching an about 1.2 kb DNA fragment containing the ORF of pgsB, which had been obtained by digesting pSPB2668 with BamHI and XhoI, to the BamHI site and the SalI site of a binary vector pSPB176 for transforming plants (JP-A-2003-432383; and van Engelen et al., Transgenic Research 4, 288-290, 1995). The multicloning site of pSPB176 is located between a CaMV35S promoter and an NOS terminator. A fragment inserted into this site is constitutionally overexpressed in plant cells under the regulation by the CaMV35S promoter.

Next, a pUC vector pSPB2670 was obtained by attaching an about 1.1 kb DNA fragment containing the ORF of pgsA, which had been obtained by digesting pSPB2667 with BamHI and SacI, to the BamHI site and the SacI site of pSPB541 (JP-A-2003-420046). The BamHI and SacI site of pSPB541 is located between a CaMV35S promoter and an NOS terminator. A fragment inserted into this site is constitutionally overexpressed in plant cells under the regulation by the CaMV35S promoter.

Next, a pUC vector pSPB2673 was obtained by attaching an about 0.45 kb DNA fragment containing the ORF of pgsC, which had been obtained by digesting pSPB2669 with KpnI and BamHI, to the KpnI site and the BamHI site of pSFL203 (JP-A-2003-420046). The KpnI and BamHI site of pSPB541 is located between a CaMV35S promoter and an NOS terminator. An insert inserted into this site is constitutionally overexpressed in plant cells under the regulation by the CaMV35S promoter.

Subsequently, an about 1.5 kb fragment containing the 35S promoter, pgsC and the NOS terminator, which had been obtained by digesting the above-described pSPB2673 with PacI, was inserted into the PacI site of the vector pSPB2672 as described above to give a binary vector pSPB2674. By PCR with the use of SEQ ID NOS:19 and 22, it was confirmed that pgsC had been inserted in the same transcriptional direction as pgsB in this binary vector. Subsequently, an about 2.1 kb fragment containing the 35S promoter, pgsA and the NOS terminator, which had been obtained by digesting the above-described pSPB2670 with AscI, was inserted into the AscI site of the vector pSPB2674 as described above to finally give a binary vector pSPB2680 constitutionally expressing pgsA, pgsB and pgsC. By PCR with the use of SEQ ID NOS:17 and 20, it was confirmed that pgsA had been inserted in the same transcriptional direction as pgsB in this binary vector.

In accordance with a publicly known method (Shimonishi et al., Shin Seibutu Kagaku Jikken no Tebiki 3, p. 122-124, Kagaku Dojin), an Agrobacterium (strain:Aglo) was transformed by using pSPB2680. Then a tobacco leaf disc was infected with this transgenic Agrobacterium having pSPB2680. By the kanamycin-screening, independent 20 transgenic tobacco lines were finally obtained.

From these transgenic tobacco lines, total RNAs were extracted by using RNeasy Plant Mini Kit (QIAGEN). Using 1 μg of the total RNAs as a template, transcription was conducted under the conditions recommended by the manufacturer (Super Script™ First-Strand Synthesis System for RT-PCR (INVITROGEN) to give cDNAs. By using these cDNAs, expression analysis was performed by RT-PCR with the use of primers of SEQ ID NOS:17 to 22. A PCR mixture (25 μl) comprised 1 μl of each cDNA, 1xEx-Taq buffer (TAKARA), 0.2 mM dNTPs, 0.2 pmol/μl portions of individual primers and 1.25 U of Ex-Taq polymerase. The PCR was conducted for 3 minutes at 94°C once and then for 28 cycles with each cycle consisting of 1 minute at 94°C, 1 minute at 53°C and 2 minutes at 72°C.

As internal standard genes for comparing expression amounts, a tobacco ubiquitin gene (NtUBQ Accession No.:U66264) and primers having the nucleotide sequences represented by SEQ ID NOS:23 and 24 (NtUBQ-FW and NtUBQ-RW) were also amplified.

SEQ ID NO:23 NtUBQ-FW
5'-ggaatgcaga tcttcgtaaa-3'
SEQ ID NO:24 NtUBQ-RW
5'- cctagaaacc accacgga-3'

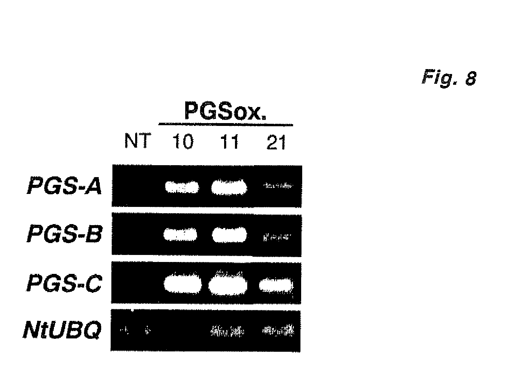

The RT-PCR products were electrophoresed in 1% agarose and fragments showing proliferation were analyzed by ethidium bromide-staining. Fig. 8 shows the results. Heterotopic expression of the three types of pgs genes was observed in transgenic lines 10, 11 and 21 of the transgenic tobacco (PGSox.). On the other hand, no pgs gene expression was observed in the nontransgenic tobacco (NT) employed as a control. Namely, transgenic tobacco plants coexpressing the three types of pgs genes were obtained. Furthermore, 4 lines expressing 2 of the three pgs genes and 4 lines expressing 1 gene were obtained.

In a crude leaf extract of a transgenic line showing the heterotopic expression of the three pgs genes, the pgs protein accumulation was immunologically confirmed by using Western blotting. The Western blotting was carried out as follows.

1. Grinding a plant while adding liquid nitrogen.

2. Adding 3 ml of 100 mM MES on ice.

3. Centrifuging at 15,000 rpm for 10 minutes.

4. Diluting the supernatant with MES to give a Western blotting sample.

5. Spotting 2 μl of the sample on a nitrocellulose membrane*1 and allowing to stand for 30 minutes.

6. Putting in a membrane bag (made of a thick plastic sheet) and shaking together with an appropriate amount (the membrane being dipped as a whole) of a saturated casein solution*2 at room temperature for 30 minutes.

7. Discarding the saturated casein solution and adding an appropriate amount of a primary antibody (serum diluted 200-fold with the saturated casein solution), followed by shaking at room temperature for 30 minutes.

8. Taking out the membrane, dipping in an appropriate amount of PBST (PBS containing 0.1% of Tween 20) and washing by shaking for 5 minutes.

9. Repeating the procedure 8 thrice.

10. Adding a secondary antibody solution [prepared by adding 0.5 ml of the saturated casein solution to 3 μl of a secondary antibody (a biotinylated universal antibody*3)] to a fresh membrane bag and shaking at room temperature for 30 minutes.

11. Taking out the membrane, dipping in an appropriate amount of PBST and washing by shaking for 5 minutes.

12. Repeating the procedure 11 thrice.

13. Preparing an avidin-biotin mixture [prepared by adding 0.5 ml of the saturated casein solution to 3 μl of avidin (ABC-AP-A*3) and 3 μl of biotin (ABC-AP-B*3)], allowing to stand for about 30 minutes, then putting into a fresh membrane bag and shaking at room temperature for 30 minutes.

14. Taking out the membrane, dipping in an appropriate amount of PBST and washing by shaking for 5 minutes.

15. Repeating the procedure 11 thrice.

16. Dipping the membrane in an appropriate amount of 100 mM Tris-HCL and washing by shaking for 5 minutes.

17. Adding a substrate solution [prepared by adding 9.5 ml of 100 mM Tris-HCl to 6 μl of the substrate 1 (BCIP-1*3), 6 μl of the substrate 2 (BCIP-2*3) and 6 μl of the substrate 3 (BCIP-3*3)] into a fresh membrane bag and allowing to stand at room temperature overnight.

*1: NITRO PLUS manufactured by MIS.

*2: Prepared by dissolving casein (milk serum) in PBS to saturation. Casein: manufactured by Wako Pure Chemical Industries.

*3: ABC System manufactured by the VECTASTAIN.

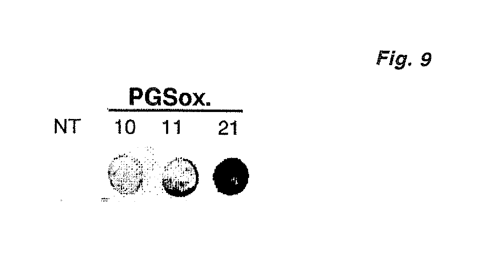

Fig. 9 shows the results. As Fig. 9 shows, pgs protein accumulation was confirmed in all of the transgenic lines 10, 11 and 21.

Example 15: Effect produced by transgenic tobacco under the existence of aluminum ion

In this Example, the transgenic tobacco lines of the present invention were examined for their greening effect under the existence of aluminum and their effect on growth inhibition.

A sheet of filter paper (Whatman, 1) was placed in each plastic dish (ϕ 85 × 13 mm) and 3 ml of 0%, 0.04% or 0.08% $AlCl_3$ (adjusted to pH 3.75 with KOH) was added thereto. Each filter paper was divided with plastic sheets (3 mm wide) into 4 equal compartments. The respective compartments were seeded with seeds of wild-type tobacco (WT) and pgsABC-transformed tobacco lines 1, 10 and 21. The dishes were then covered and sealed with surgical tape, followed by incubation at 25°C for 10 days under continuous white light irradiation.

On the 6th day, 1 ml of 0%, 0.04% or 0.08% $AlCl_3$ was further added to prevent dryness. In the $AlCl_3$-free control, all plants extended their roots and showed no difference in their cotyledons. Under the existence of 0.08% $AlCl_3$,

there was no difference among the lines because their roots were significantly prevented from extending, and most of their cotyledons turned brown.

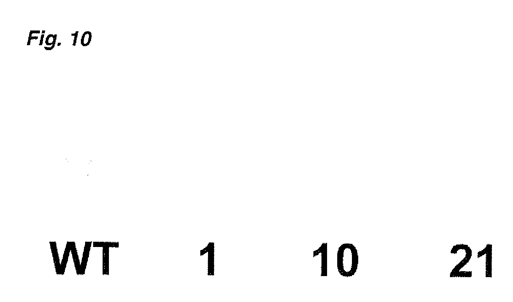

However, there was a difference in cotyledons under the existence of 0.04% $AlCl_3$. The representative gemmation in this case is shown in Fig. 10. The greening of cotyledons was strongly prevented in WT, whereas cotyledons greened in the transgenic tobacco lines 1, 10 and 21 of the present invention.

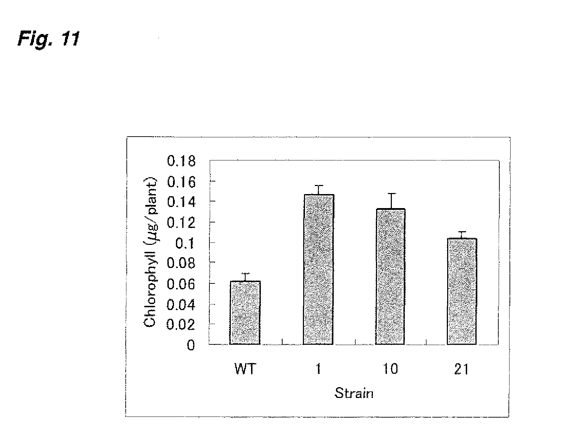

To determine the chlorophyll content, 12 gemmated plants were randomly harvested from the 0.04% $AlC1_3$ compartment and then homogenized in the presence of 120 μl acetone. After centrifugation at 20,000 × g for 5 minutes, the supernatants were measured for their absorbance at 652 nm using a spectrophotometer. The absorption coefficient was set to 34.5 $mg^{-1}mlcm^{-1}$ and the chlorophyll concentration was determined. As shown in Fig. 11, the transformants had significantly higher chlorophyll contents than the wild-type ($P<0.05$), indicating that $AlCl_3$-induced growth inhibition was relieved.

## Claims

1. A method for preparing a transgenic plant for producing polyglutamic acid comprising:

   introducing a nucleic acid encoding the polyglutamic acid synthase A (pgsA), a nucleic acid encoding the polyglutamic acid synthase B (pgsB) and a nucleic acid encoding the polyglutamic acid synthase C (pgsC) into a plant.

2. The method of Claim 1 wherein
   the polyglutamic acid synthase A has the amino acid sequence of SEQ ID NO:1 or an amino acid sequence wherein one or more amino acid residues are deleted from, added to or substituted in SEQ ID NO:1, the polyglutamic acid synthase B has the amino acid sequence of SEQ ID NO:3 or an amino acid sequence wherein one or more amino acid residues are deleted from, added to or substituted in SEQ ID NO:3, the polyglutamic acid synthase C has the amino acid sequence of SEQ ID NO:5 or an amino acid sequence wherein one or more amino acid residues are deleted from, added to or substituted in SEQ ID NO:5; and
   three types of the polyglutamic acid synthase, A, B and C, together, have the function to produce polyglutamic acid.

3. The method of Claim 1 wherein
   the polyglutamic acid synthase A has the amino acid sequence of SEQ ID NO:1 or an amino acid sequence which is at least 70% identical with SEQ ID NO:1, the polyglutamic acid synthase B has the amino acid sequence of SEQ ID NO:3 or an amino acid sequence which is at least 70% identical with SEQ ID NO:3, and the polyglutamic acid synthase C has the amino acid sequence of SEQ ID NO:5 or an amino acid sequence which is at least 70% identical with SEQ ID NO:5; and
   three types of the polyglutamic acid synthase, A, B and C, together, have the function to produce polyglutamic acid.

4. The method of any one of Claims 1-3 wherein the plant is infected with Agrobacterium containing a vector inserted with one or more nucleic acids selected from a nucleic acid encoding the polyglutamic acid synthase A (pgsA), a nucleic acid encoding the polyglutamic acid synthase B (pgsB) and a nucleic acid encoding the polyglutamic acid synthase C (pgsC) to introduce the nucleic acids into the plant.

5. The method of Claim 4 wherein introduction of the three types of the nucleic acids, pgsA, pgsB and pgsC is performed by any one of the following steps a) - c):

   a) infecting the plant with Agrobacterium containing a single vector inserted with three types of nucleic acids, pgsA, pgsB and pgsC;
   b) infecting the plant with three types of Agrobacterium each of them containing a distinct vector inserted with pgsA, pgsB or pgsC, respectively; or
   c) infecting the plant with one or two types of Agrobacterium each of them containing a distinct vector inserted with pgsA, pgsB or pgsC, respectively to prepare transgenic plants having one or two nucleic acids selected from pgsA, pgsB and pgsC, and crossing the prepared transgenic plants.

6. The method of Claim 4 or 5 wherein the plant is infected with Agrobacterium by a method selected from the group consisting of the leaf disc method, the decompression-humectation method and the direct injection method.

7. The method of any one of Claims 1-6 wherein the plant is selected from the group consisting of tobacco, Arabidopsis thaliana, rice, soybean and bird's-foot trefoil.

**8.** A transgenic plant for producing polyglutamic acid prepared by the method of any one of claims 1-7.

**9.** The transgenic plant of Claim 8, wherein the plant is in the phase of adult, seed or callus.

**10.** The transgenic plant of Claim 8 or 9, wherein the plant is tobacco or Arabidopsis thaliana.

```
   1 CAAACGATGA AAAAAGAACT GAGCTTTCAT GAAAAGCTGC TAAAGCTGAC AAAACAGCAA AAAAAGAAAA CCAATAAGCA CGTATTTATT
   1       M    K  K  E  L    S  F  H    E  K  L    L  K  L  T    K  Q  Q    K  K  K    T  N  K  H    V  F  I
  91 GCCATTCCGA TCGTTTTTGT CCTTATGTTC GCTTTCATGT GGGCGGGAAA AGCGGAAACG CCGAAGGTCA AAACGTATTC TGACGACGTA
  29 A  I  P    I  V  F  V    L  M  F    A  F  M    W  A  G  K    A  E  T    P  K  V    K  T  Y  S    D  D  V
 181 CTCTCAGCCT CATTTGTAGG CGATATTATG ATGGGACGCT ATGTTGAAAA AGTAACGGAG CAAAAAGGGG CAGACAGTAT TTTTCAATAT
  59 L  S  A    S  F  V  G    D  I  M    M  G  R    Y  V  E  K    V  T  E    Q  K  G    A  D  S  I    F  Q  Y
 271 GTTGAACCGA TCTTTAGAGC CTCGGATTAT GTAGCAGGAA ACTTTGAAAA CCCGGTAACC TATCAAAAGA ATTATAAACA AGCAGATAAA
  89 V  E  P    I  F  R  A    S  D  Y    V  A  G    N  F  E  N    P  V  T    Y  Q  K    N  Y  K  Q    A  D  K
 361 GAGATTCATC TGCAGACGAA TAAGGAATCA GTGAAAGTCT TGAAGGATAT GAATTTCACG GTTCTCAACA GCGCAAACAA CCACGCAATG
 119 E  I  H    L  Q  T  N    K  E  S    V  K  V    L  K  D  M    N  F  T    V  L  N    S  A  N  N    H  A  M
 451 GATTACGGCG TTCAGGGCAT GAAAGATACG CTTGGAGAAT TTGCGAAGCA AAACCTTGAT ATCGTTGGAG CGGGATACAG CTTAAGTGAT
 149 D  Y  G    V  Q  G  M    K  D  T    L  G  E    F  A  K  Q    N  L  D    I  V  G    A  G  Y  S    L  S  D
 541 GCGAAAAAGA AAATTTCGTA CCAAAAAGTC AACGGGGTAA CGATTGCGAC GCTTGGCTTT ACCGATGTGT CCGGGAAAGG TTTCGCGGCT
 179 A  K  K    K  I  S  Y    Q  K  V    N  G  V    T  I  A  T    L  G  F    T  D  V    S  G  K  G    F  A  A
 631 AAAAAAAATA CGCCGGGCGT GCTGCCCGCA GATCCTGAAA TTTTCATCCC TATGATTTCA GAAGCGAAAA AACATGCTGA CATTGTTGTT
 209 K  K  N    T  P  G  V    L  P  A    D  P  E    I  F  I  P    M  I  S    E  A  K    K  H  A  D    I  V  V
 721 GTGCAGTCAC ACTGGGGCCA AGAGTATGAC AATGATCCAA ACGACCGCCA GCGCCAGCTT GCAAGAGCCA TGTCTGATGC GGGAGCTGAC
 239 V  Q  S    H  W  G  Q    E  Y  D    N  D  P    N  D  R  Q    R  Q  L    A  R  A    M  S  D  A    G  A  D
 811 ATCATCGTCG GCCATCATCC GCACGTCTTA GAACCGATTG AAGTATATAA CGGAACCGTC ATTTTCTACA GCCTCGGCAA CTTTGTCTTT
 269 I  I  V    G  H  H  P    H  V  L    E  P  I    E  V  Y  N    G  T  V    I  F  Y    S  L  G  N    F  V  F
 901 GACCAAGGCT GGACGAGAAC AAGAGACAGT GCACTGGTTC AGTATCACCT GAAGAAAAAT GGAACAGGCC GCTTTGAAGT GACACCGATC
 299 D  Q  G    W  T  R  T    R  D  S    A  L  V    Q  Y  H  L    K  K  N    G  T  G    R  F  E  V    T  P  I
 991 GATATCCATG AAGCGACACC TGCACCTGTG AAAAAAGACA GCCTTAAACA GAAAACCATT ATTCGCGAAC TGACGAAAGA CTCTAATTTC
 329 D  I  H    E  A  T  P    A  P  V    K  K  D    S  L  K  Q    K  T  I    I  R  E    L  T  K  D    S  N  F
1081 GCTTGGAAAG TAGAAGACGG AAAACTGACG TTTGATATTG ATCATAGTGA CAAACTAAAA TCTAAATAA
 359 A  W  K    V  E  D  G    K  L  T    F  D  I    D  H  S  D    K  L  K    S  K
```

*Fig. 1*

```
   1 GCAATGTGGT TACTCATTAT AGCCTGTGCT GTCATACTGG TCATCGGAAT ATTAGAAAAA CGACGACATC AGAAAAACAT TGATGCCCTC
   1    M  W   L  L  I  I   A  C  A   V  I  L   V  I  G  I   L  E  K   R  R  H   Q  K  N  I   D  A  L
  91 CCTGTTCGGG TGAATATTAA CGGCATCCGC GGAAAATCGA CTGTGACAAG GCTGACAACC GGAATATTAA TAGAAGCCGG TTACAAGACT
  30 P  V  R   V  N  I  N   G  I  R   G  K  S   T  V  T  R   L  T  T   G  I  L   I  E  A  G   Y  K  T
 181 GTTGGAAAAA CAACAGGAAC AGATGCAAGA ATGATTTACT GGGACACACC GGAGGAAAAG CCGATTAAAC GGAAACCTCA GGGGCCGAAT
  60 V  G  K   T  T  G  T   D  A  R   M  I  Y   W  D  T  P   E  E  K   P  I  K   R  K  P  Q   G  P  N
 271 ATCGGAGAGC AAAAAGAAGT CATGAGAGAA ACAGTAGAAA GAGGGGCTAA CGCGATTGTC AGTGAATGCA TGGCTGTTAA CCCAGATTAT
  90 I  G  E   Q  K  E  V   M  R  E   T  V  E   R  G  A  N   A  I  V   S  E  C   M  A  V  N   P  D  Y
 361 CAAATCATCC TTCAGGAAGA ACTTCTGCAG GCCAATATCG GCGTCATTGT GAATGTTTTA GAAGACCATA TGGATGTCAT GGGGCCGACG
 120 Q  I  I   L  Q  E  E   L  L  Q   A  N  I   G  V  I  V   N  V  L   E  D  H   M  D  V  M   G  P  T
 451 CTTGATGAAA TTGCAGAAGC GTTTACCGCT ACAATTCCTT ATAATGGCCA TCTTGTCATT ACAGATAGTG AATATACCGA GTTCTTTAAA
 150 L  D  E   I  A  E  A   F  T  A   T  I  P   Y  N  G  H   L  V  I   T  D  S   E  Y  T  E   F  F  K
 541 CAAAAAGCAA AAGAACGAAA CACAAAAGTC ATCATTGCTG ATTACTCAAA AATTACAGAT GAGTATTTAC GTAAATTTGA ATACATGGTA
 180 Q  K  A   K  E  R  N   T  K  V   I  I  A   D  Y  S  K   I  T  D   E  Y  L   R  K  F  E   Y  M  V
 631 TTCCCTGATA ACGCTTCTCT GGCGCTGGGT GTGGCTCAAG CACTCGGCAT TGACGAAGAA ACAGCATTTA AGGGAATGCT GAATGCGCCG
 210 F  P  D   N  A  S  L   A  L  G   V  A  Q   A  L  G  I   D  E  E   T  A  F   K  G  M  L   N  A  P
 721 CCAGATCCGG GAGCAATGAG AATTCTTCCG CTGATCAGTC CGAGCGAGCC TGGGCACTTT GTTAATGGGT TTGCCGCAAA CGACGCTTCT
 240 P  D  P   G  A  M  R   I  L  P   L  I  S   P  S  E  P   G  H  F   V  N  G   F  A  A  N   D  A  S
 811 TCTACTTTGA ATATATGGAA ACGTGTAAAA GAAATCGGTT ACCCGACCGA TGATCCGATC ATCATCATGA ACTGCCGCGC AGACCGTGTC
 270 S  T  L   N  I  W  K   R  V  K   E  I  G   Y  P  T  D   D  P  I   I  I  M   N  C  R  A   D  R  V
 901 GATCGGACAC AGCAATTCGC AAATGACGTA TTGCCTTATA TTGAAGCAAG TGAACTGATC TTAATCGGTG AAACAACAGA ACCGATCGTA
 300 D  R  T   Q  Q  F  A   N  D  V   L  P  Y   I  E  A  S   E  L  I   L  I  G   E  T  T  E   P  I  V
 991 AAAGCCTATG AAGAAGGCAA AATTCCTGCA GACAAACTGC ATGATCTAGA GTATAAGTCA ACAGATGAAA TTATGGAATT GTTAAAGAAA
 330 K  A  Y   E  E  G  K   I  P  A   D  K  L   H  D  L  E   Y  K  S   T  D  E   I  M  E  L   L  K  K
1081 AGAATGCACA ACCGTGTCAT ATATGGCGTC GGCAATATTC ATGGTGCCGC AGAGCCTTTA ATTGAAAAAA TCCACGAATA CAAGGTAAAG
 360 R  M  H   N  R  V  I   Y  G  V   G  N  I   H  G  A  A   E  P  L   I  E  K   I  H  E  Y   K  V  K
1171 CAGCTCGTAA GCTAG
 390 Q  L  V   S
```

*Fig. 2*

```
  1 AAATGCAGAC ATGTTCGGAT CAGATTTATA CATCGCACTA ATTTTAGGTG TACTACTCAG TTTAATTTTT GCGGAAAAAA CAGGGATCGT
  1    N  A  D   M  F  G   S  D  L   Y  I  A  L   I  L  G   V  L  L   S  L  I  F   A  E  K   T  G  I
 91 GCCGGCAGGA CTGGTTGTAC CGGGATATTT AGGACTTGTG TTTAATCAGC CGGTCTTTAT TTTACTTGTT TTGCTAGTGA GCTTGCTCAC
 31 V  P  A  G   L  V  V   P  G  Y   L  G  L  V   F  N  Q   P  V  F   I  L  L  V   L  L  V   S  L  L
181 GTATGTTATC GTGAAATACG GTTTATCCAA ATTTATGATT TTGTACGGAC GCAGAAAATT TGCTGCCATG CTGATAACAG GGATCGTCCT
 61 T  Y  V  I   V  K  Y   G  L  S   K  F  M  I   L  Y  G   R  R  K   F  A  A  M   L  I  T   G  I  V
271 AAAAATCGCG TTTGATTTTC TATACCCGAT TGCACCATTT GAAATCGCAG AATTTCGAGG AATCGGCATC ATCGTGCCAG GTTTAATTGC
 91 L  K  I  A   F  D  F   L  Y  P   I  A  P  F   E  I  A   E  F  R   G  I  G  I   I  V  P   G  L  I
361 CAATACCATT CAGAAACAAG GTTTAACCAT TACGTTCGGA AGCACGCTGC TATTGAGCGG AGCGACCTTT GCTATCATGT TTGTTTACTA
121 A  N  T  I   Q  K  Q   G  L  T   I  T  F  G   S  T  L   L  L  S   G  A  T  F   A  I  M   F  V  Y
451 CTTAATTTAA T
151 Y  L  I
```

*Fig. 3*

100 倍
10 倍
1 倍

*Fig. 4*

**Western blotting (supernatant) of tobacco leaf sections having the PGA synthases introduced therein by the direct injection method**

100 倍
10 倍
1 倍

*Fig. 5*

**Western blotting (precipitate) of tobacco leaf sections having the PGA synthases introduced therein by the direct injection method**

BSA 2mg

BSA PGA

0. 025mg Conjugation

0. 25mg

2. 5mg

*Fig. 6*

**Western blotting with the use of the crosslinked PGA-BSA**

pgsB
pgsC
pgsA
Ywtc
pgdS

Fig. 7

Fig. 8

PGSox.
NT
10
11
21
PGS-A
PGS-B
PGS-C
NtUBQ

*Fig. 9*

PGSox.
NT
10
11
21

*Fig. 10*

WT 1 10 21

*Fig. 11*

0.18
0.16
0.14
0.12
0.1
0.08
0.06
0.04
0.02
0
Chlorophyll (μg/plant)
WT
1
10
21
Strain

**INTERNATIONAL SEARCH REPORT**

International application No.
PCT/JP2006/304083

A. CLASSIFICATION OF SUBJECT MATTER
*A01H5/00*(2006.01), *C12N15/09*(2006.01)

According to International Patent Classification (IPC) or to both national classification and IPC

B. FIELDS SEARCHED

Minimum documentation searched (classification system followed by classification symbols)
*A01H5/00*(2006.01), *C12N15/09*(2006.01)

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
BIOSIS/WPIDS/MEDLINE/CA(STN), JMEDPlus(JDream2), JST7580(JDream2), JSTPlus(JDream2)

C. DOCUMENTS CONSIDERED TO BE RELEVANT

| Category* | Citation of document, with indication, where appropriate, of the relevant passages | Relevant to claim No. |
|---|---|---|
| X | JP 2001-017182 A (NAGASE & CO LTD), 23 January, 2001 (23.01.01), Particularly, Par. Nos. [0024], [0041]; sequence No.1 to 4 (Family: none) | 1-10 |
| A | ASHIUCHI M. et al, A poly-γ-glutamate synthetic system of *Bacillus subtilis* IFO 3336: gene cloning and biochemical analysis of poly-γ-glutamate produced by *Escherichia coli* clone cells., Biochem.Biophys.Res.Commun., 1999, Vol.263, No.1, pages 6 to 12 | 1-10 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 May, 2006 (17.05.06) | 23 May, 2006 (23.05.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

INTERNATIONAL SEARCH REPORT

International application No. PCT/JP2006/304083

C (Continuation). DOCUMENTS CONSIDERED TO BE RELEVANT

| Category* | Citation of document, with indication, where appropriate, of the relevant passages | Relevant to claim No. |
|---|---|---|
| P,X | Biosynthesis of poly-γ-glutamic acid in plants: transient expression of poly-γ-glutamate synthetase complex in tobacco leaves., Journal of bioscience and bioengineering, Oct.2005, Vol.100, No.4, p.443-8 | 1-10 |
| P,X | Hanako IIDA et al., "poly-γ-glutamic acid o Seisan suru Transgenic Shokubutsu no Sakushutsu", The Society for Biotechnology, Japan Taikai Koen Yoshishu, Sep.2005, Vol.2005, page 65 | 1-10 |
| P,X | Hanako IIDA et al., "poly-γ-glutamic acid o Seisan suru Transgenic Shokubutsu no Sakushutsu", Japan Society for Bioscience, Biotechnology, and Agrochemistry Kansai Shibu Koenkai Koen Yoshishu, Jul.2005, Vol.440th, page 15 | 1-10 |
| P,X | Hanako IIDA et al., "PGA Gosei Idenshi ni yoru Keishitsu Tenkan Shokubutsu Sakushutsu", Nippon Nogei Kagakukai Taikai Koen Yoshishu, 05 March, 2005 (05.03.05), Vol.2005, page 152 | 1-10 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2003420046 A
- JP 2003432383 A
- EP 0525882 A


### Non-patent literature cited in the description


- **ASHIUCHI, M. ; NAWA, C. ; KAMEI, T. ; SONG, J.J. ; HONG, S.P. ; SUNG, M.H. ; SODA, K. ; MISONO, H.** Physiological and biochemical characteristics of poly-γ-glutamate synthetase complex of Bacillus subtilis. *Eur. J. Biochem.,* 2001, vol. 268, 5321-5328
- **HORSCH, R.B. ; FRY, J. ; HOFFMANN, N. ; NEIDERMERYER, J. ; ROGERS, S.G. ; FRALEY, R.T.** Leaf disc transformation. *Plant Mol. Biol. Mannual,* 1988, vol. A5, 1-9
- **JOHANSEN, L.K. ; CARRINGTON, J.C.** Silencing on the spot, Induction and suppression of RNA silencing in the Agrobacterium-mediated transient expression system. *Plant Physiol.,* 2001, vol. 126, 930-938
- **YAMAGUCHI, F. ; OGAWA, Y. ; KIKUCHI, M. ; YUASA, K. ; MOTAI, H.** Detection of γ-polyglutamic acid (γ-PGA) by SDS-PAGE. *Biosci. Biotech. Biochem.,* 1996, vol. 60, 255-258
- **YENOFSKY, R.L. ; FINE, M. ; PELLOW, J.W.** A mutant neomycin phosphotransferase II gene reduces the resistance of transformants to antibiotic selection pressure. *Proc. Natl. Acad. Sci. USA,* 1990, vol. 87, 3455-3439
- **ASHIUCHI et al.** A poly-γ-glutamate synthetic system of Bacillus subtilis IFO 3336: gene cloning and biochemical analysis of poly-γ-glutamate produced by Escherichia coli clone cells. *Bioche. Biophys. Res. Commun.,* 1999, vol. 263, 387-393
- **ASHIUCHI ; MISONO.** Biochemistry and molecular genetics of poly-γ-glutamate synthesis. *Appl. Microbiol. Biotechnol.,* 2002, vol. 59, 9-14
- **SHIMONISHI et al.** Shin Seibutu Kagaku Jikken no Tebiki. Kagaku Dojin, 1996, vol. 3, 122-124
- **HIEI et al.** *Plant J.,* 1994, vol. 6, 271-282
- **KOMARI et al.** *Plant J.,* 1996, vol. 10, 165-174
- **VAN ENGELEN et al.** *Transgenic Research,* 1995, vol. 4, 288-290
- **JEFFERSON et al.** GUS fusion:β-glucosidase as a sensitive and versatile gene fusion marker in higher plants. *EMBO J.,* 1987, vol. 6, 3901-3907
- **MATSUMOTO ; MACHIDA.** Shokubutsu Keishitsu Tenkan-ho. *Gendai Kagaku,* 1990
- **ARAKI.** Genatsu Shinjun-ho ni yoru Keishitsutenkan, Saibo Kogaku. *Shokubutsu Saibo Kogaku Shirizu,* 2001, vol. 15, 109-113
- **OSUMI.** Agurobakuteriumu Chokusetsu Chunyu-ho, Saibo Kogaku. *Shokubutsu Saibo Kogaku Shirizu,* 2001, vol. 15, 105-108
- **NEEDLEMAN, S.B. ; WUNSCH, C.D.** *J. Mol. Biol.,* 1970, vol. 48, 443-453
- **HENIKOFF, S. ; HENIKOFF, J.G.** *Porc. Natl. Acad. Sci. USA,* 1992, vol. 89, 10915-10919
- **ALTSCHUL et al.** *Nucl. Acids. Res.,* 1997, vol. 25, 3389-3402
- **SAMBROOK et al.** Molecular Cloning: A Laboratory Manual. Cold Spring Harbor Laboratory Press, 2001
- **DEVEREUX et al.** *Nucl. Acids. Res.,* 1984, vol. 12, 387
- **GRIBSKOV ; BURGESS.** *Nucl. Acids Res.,* 1986, vol. 14, 6745
- Atlas of Polypeptide Sequence and Structure. National Biomedical Research Foundation, 1979, 353-358
- **WOOTTON ; FEDERHEN.** Analysis of compositionally biased regions in sequence databases. *Methods Enzymol.,* 1996, vol. 266, 554-71
- **SAMBROK, J. ; RUSSELL, D.W.** Molecular Cloning: A Laboratory Manual. Cold Spring Harbor Laboratory Press, 2001
- **SHIMONISHI et al.** Shin Seibutsu Kagaku Jikken no Tebiki. Kagaku Dojin, 1996, vol. 3, 122-124
- **ARAKI.** Genatsu Shinjun-ho ni yoru Keishitsu Tenkan, Saibo Kogaku. *Shokubutsu Saibo Kogaku Shirizu,* 2001, vol. 15, 109-113
- **SAIKI R.K. et al.** *Science,* 1985, vol. 230, 1350-1354

- **WU D.Y. et al.** *Genomics,* 1989, vol. 4, 560-569
- **WALKER G.T. et al.** *Proc. Natl. Acad. Sic. USA,* 1992, vol. 89, 392-396
- **GUATELLI J.C.** *Proc. Natl. Acad. Sci. USA,* 1990, vol. 87, 1874-1878
- **LIZARDI et al.** *Bio Technology,* 1988, vol. 6, 1197-1202
- Hybridoma Techniques. Cold Spring Harbor Laboratory, 1980
- **SHIMONISHI et al.** Shin Seibutu Kagaku Jikken no Tebiki. Kagaku Dojin, vol. 3, 122-124